# EUROPEAN PATENT APPLICATION

(11) **EP 3 960 862 A1**
(43) Date of publication of application: **02.03.2022**
(21) Application number: 20193971.7
(22) Date of filing: 01.09.2020
(51) Int. Cl.: C12N 15/115, C12Q 1/6825, C12N 15/63

(54) **RNA-APTAMER-SENSORS**

(71) Applicant: SenseUp GmbH, 52428 Jülich (DE)
(72) Inventor: Flachbart, Lion, 40597 Düsseldorf (DE); Mahr, Regina, 50226 Frechen (DE); Schaumann, Georg, 40223 Düsseldorf (DE)
(74) Representative: Stolmár & Partner Patentanwälte PartG mbB

(57) **Abstract**

The present invention relates to a method for optimizing the production of a RNA sequence of interest in a cell, comprising the steps of a) introducing into a plurality of cells a vector capable of expressing a heterologous RNA of interest, wherein said RNA is tagged with a RNA tag comprising an aptamer capable of stabilizing a fluorophore and a scaffold capable of stabilizing the aptamer; b) culturing the cells in a culture medium under conditions that allow expression of the heterologous RNA of interest; c) adding said fluorophore to the culture medium; and d) identifying those cells that show the highest intensity of fluorescence and therefore a high expression of the RNA of interest.

SEQUENCE LISTING

<110>  SenseUp GmbH

<120>  RNA sensors

<130>  38917-SSU-P-EP

<160>  78

<170>  PatentIn version 3.5

<210>  1
<211>  29
<212>  RNA
<213>  Artificial

<220>
<223>  Aptamer

<400>  1
agguucgaag cuuuugcuug gacgaaccg                                          29


<210>  2
<211>  23
<212>  RNA
<213>  Artificial

<220>
<223>  Aptamer

<400>  2
gaagggacgg ugcggagagg aga                                                23


<210>  3
<211>  25
<212>  RNA
<213>  Artificial

<220>
<223>  Aptamer

<400>  3
cgagggagug gugaggauga ggcga                                              25


<210>  4
<211>  25
<212>  RNA
<213>  Artificial

<220>
<223>  Aptamer

<400>  4
cgagggagug gugaggauga ggcga                                              25


<210>  5
<211>  25
<212>  RNA

<213>  Artificial

<220>
<223>  Aptamer

<400>  5
cgagggagug gugaggauga ggcga                                          25


<210>  6
<211>  98
<212>  RNA
<213>  Artificial

<220>
<223>  Aptamer

<400>  6
gacgcaacug aaugaaaugg ugaaggacgg guccaggugu ggcugcuucg gcagugcagc      60

uuguugagua gagugugagc uccguaacua gucgcguc                            98


<210>  7
<211>  95
<212>  RNA
<213>  Artificial

<220>
<223>  Aptamer

<400>  7
gauguaacug aaugaaaugg ugaaggacgg guccaguagg cugcuucggc agccuacuug      60

uugaguagag ugugagcucc guaacuaguu acauc                               95


<210>  8
<211>  47
<212>  RNA
<213>  Artificial

<220>
<223>  Aptamer

<400>  8
agacggucgg guccagauau ucguaucugu cgaguagagu gugggcu                  47


<210>  9
<211>  65
<212>  RNA
<213>  Artificial

<220>
<223>  Scaffold


<220>
<221>  misc_feature
<223>  tRNALys

<220>

<221>  misc_feature
<222>  (28)..(31)
<223>  Insertion site for aptamer

<400>  9
gcccggauag cucagucggu agagcagnnn ncggguccag gguucaaguc ccuguucggg      60

cgcca                                                                  65


<210>  10
<211>  86
<212>  RNA
<213>  Artificial

<220>
<223>  Scaffold


<220>
<221>  misc_feature
<222>  (23)..(26)
<223>  Insertion site

<400>  10
ugccuggcga ccauagcgau ugnnnncaau uagcgccgau gguagugugg gguuucccca      60

ugugagagua ggacaucgcc aggcau                                           86


<210>  11
<211>  62
<212>  RNA
<213>  Artificial

<220>
<223>  Scaffold


<220>
<221>  misc_feature
<223>  F29

<220>
<221>  misc_feature
<222>  (19)..(22)
<223>  Insertion site for aptamer

<220>
<221>  misc_feature
<222>  (43)..(46)
<223>  Insertion site for aptamer

<400>  11
uugucacgug uaugugggnn nncccacaua cuuuguugau ccnnnnggau caaucauggc      60

aa                                                                     62


<210>  12
<211>  62
<212>  RNA
<213>  Artificial

<220>
<223>  Scaffold

<220>
<221>  misc_feature
<223>  F30

<220>
<221>  misc_feature
<222>  (19)..(22)
<223>  Insertion site for aptamer

<220>
<221>  misc_feature
<222>  (43)..(46)
<223>  Insertion site for aptamer

<400>  12
uugccaugug uaugugggnn nncccacaua cucugaugau ccnnnnggau cauucauggc      60

aa                                                                    62

<210>  13
<211>  28
<212>  RNA
<213>  Artificial

<220>
<223>  Aptamer

<400>  13
cgaggaagga ggucugagga ggucacug                                        28

<210>  14
<211>  52
<212>  RNA
<213>  Artificial

<220>
<223>  Aptamer

<400>  14
agacggucgg guccaggugc acaaaugugg ccuguugagu agcguguggg cu             52

<210>  15
<211>  52
<212>  RNA
<213>  Artificial

<220>
<223>  Aptamer

<400>  15
agacggucgg guccaguccc aacgauguug gcuguugagu aguguguggg cu            52

<210>  16
<211>  29

<212>  RNA
<213>  Artificial

<220>
<223>  Aptamer

<400>  16
aaggacgggu ccguugagua gagugugag                                          29


<210>  17
<211>  90
<212>  RNA
<213>  Artificial

<220>
<223>  Scaffold and aptamer

<400>  17
gcccggauag cucagucggu agagcagagg uucgaagcuu uugcuuggac gaaccgcggg        60

uccaggguuc aagucccugu ucgggcgcca                                         90


<210>  18
<211>  84
<212>  RNA
<213>  Artificial

<220>
<223>  Scaffold + aptamer

<400>  18
gcccggauag cucagucggu agagcaggaa gggacggugc ggagaggaga cggguccagg        60

guucaagucc cuguucgggc gcca                                               84


<210>  19
<211>  86
<212>  RNA
<213>  Artificial

<220>
<223>  RNA scaffold + aptamar

<400>  19
gcccggauag cucagucggu agagcagcga gggaguggug aggaugaggc gacgggucca        60

ggguucaagu cccuguucgg gcgcca                                             86


<210>  20
<211>  86
<212>  RNA
<213>  Artificial

<220>
<223>  RNA scaffold + aptamer

<400>  20
gcccggauag cucagucggu agagcagcga gggaguggug aggaugaggc gacgggucca        60

ggguucaagu cccuguucgg gcgcca                                          86

<210>  21
<211>  86
<212>  RNA
<213>  Artificial

<220>
<223>  RNA scaffold + aptamer

<400>  21
gcccggauag cucagucggu agagcagcga gggaguggug aggaugaggc gacgggucca      60

ggguucaagu cccuguucgg gcgcca                                          86

<210>  22
<211>  159
<212>  RNA
<213>  Artificial

<220>
<223>  RNA scaffold + aptamer

<400>  22
   gcccggauag cucagucggu agagcaggac gcaacugaau gaaaugguga aggacggguc      60
   cagguguggc ugcuucggca gugcagcuug uugaguagag ugugagcucc guaacuaguc     120
   gcguccgggu ccaggguuca agucccuguu cgggcgcca                           159

<210>  23
<211>  156
<212>  RNA
<213>  Artificial

<220>
<223>  RNA scaffold + aptamer

<400>  23
gcccggauag cucagucggu agagcaggau guaacugaau gaaaugguga aggacggguc      60

caguaggcug cuucggcagc cuacuuguug aguagagugu gagcuccgua acuaguuaca     120

uccgggucca ggguucaagu cccuguucgg gcgcca                              156

<210>  24
<211>  108
<212>  RNA
<213>  Artificial

<220>
<223>  RNA scaffold + aptamer

<400>  24
gcccggauag cucagucggu agagcagaga cggucggguc cagauauucg uaucugucga      60

guagagugug ggcucggguc caggguucaa gucccuguuc gggcgcca                 108

<210>  25
<211>  89
<212>  RNA
<213>  Artificial

<220>
<223>  RNA scaffold + aptamer

<400>  25
gcccggauag cucagucggu agagcagcga ggaaggaggu cugaggaggu cacugcgggu      60

ccaggguuca agucccuguu cgggcgcca                                        89


<210>  26
<211>  113
<212>  RNA
<213>  Artificial

<220>
<223>  RNA scaffold + aptamer

<400>  26
gcccggauag cucagucggu agagcagaga cggucggguc caggugcaca aauguggccu      60

guugaguagc gugugggcuc ggguccaggg uucaaguccc uguucgggcg cca            113


<210>  27
<211>  113
<212>  RNA
<213>  Artificial

<220>
<223>  RNA scaffold + aptamer

<400>  27
gcccggauag cucagucggu agagcagaga cggucggguc cagucccaac gauguuggcu      60

guugaguagu gugugggcuc ggguccaggg uucaaguccc uguucgggcg cca            113


<210>  28
<211>  90
<212>  RNA
<213>  Artificial

<220>
<223>  RNA scaffold + aptamer

<400>  28
gcccggauag cucagucggu agagcagaag gacggguccg uugaguagag ugugagcggg      60

uccaggguuc aagucccugu ucgggcgcca                                       90


<210>  29
<211>  111
<212>  RNA
<213>  Artificial

<220>
<223>  RNA scaffold + aptamer

<400>  29
ugccuggcga ccauagcgau ugagguucga agcuuuugcu uggacgaacc gcaauuagcg      60

ccgaugguag ugugggguuu ccccauguga gaguaggaca ucgccaggca u               111


<210>  30
<211>  105
<212>  RNA
<213>  Artificial

<220>
<223>  RNA scaffold + aptamer

<400>  30
ugccuggcga ccauagcgau uggaagggac ggugcggaga ggagacaauu agcgccgaug      60

guaguguggg guuuccccau gugagaguag gacaucgcca ggcau                     105


<210>  31
<211>  107
<212>  RNA
<213>  Artificial

<220>
<223>  RNA scaffold + aptamer

<400>  31
ugccuggcga ccauagcgau ugcgagggag uggugaggau gaggcgacaa uuagcgccga      60

ugguagugug ggguuucccc augugagagu aggacaucgc caggcau                   107


<210>  32
<211>  107
<212>  RNA
<213>  Artificial

<220>
<223>  RNA scaffold + aptamer

<400>  32
ugccuggcga ccauagcgau ugcgagggag uggugaggau gaggcgacaa uuagcgccga      60

ugguagugug ggguuucccc augugagagu aggacaucgc caggcau                   107


<210>  33
<211>  107
<212>  RNA
<213>  Artificial

<220>
<223>  RNA scaffold + aptamer

<400>  33
ugccuggcga ccauagcgau ugcgagggag uggugaggau gaggcgacaa uuagcgccga      60

ugguagugug ggguuucccc augugagagu aggacaucgc caggcau                   107

<210>  34
<211>  180
<212>  RNA
<213>  Artificial

<220>
<223>  RNA scaffold + aptamer

<400>  34
ugccuggcga ccauagcgau uggacgcaac ugaaugaaau ggugaaggac ggguccaggu      60

guggcugcuu cggcagugca gcuuguugag uagaguguga gcuccguaac uagucgcguc      120

caauuagcgc cgaugguagu gugggguuuc cccaugugag aguaggacau cgccaggcau      180


<210>  35
<211>  177
<212>  RNA
<213>  Artificial

<220>
<223>  RNA scaffold + aptamer

<400>  35
ugccuggcga ccauagcgau uggauguaac ugaaugaaau ggugaaggac ggguccagua      60

ggcugcuucg gcagccuacu uguugaguag agugugagcu ccguaacuag uuacauccaa      120

uuagcgccga ugguagugug ggguuucccc augugagagu aggacaucgc caggcau      177


<210>  36
<211>  129
<212>  RNA
<213>  Artificial

<220>
<223>  RNA scaffold + aptamer

<400>  36
ugccuggcga ccauagcgau ugagacgguc ggguccagau auucguaucu gucgaguaga      60

gugugggcuc aauuagcgcc gaugguagug ugggguuucc ccaugugaga guaggacauc      120

gccaggcau                                                              129


<210>  37
<211>  110
<212>  RNA
<213>  Artificial

<220>
<223>  RNA scaffold + aptamer

<400>  37
ugccuggcga ccauagcgau ugcgaggaag gaggucugag gaggucacug caauuagcgc      60

cgaugguagu gugggguuuc cccaugugag aguaggacau cgccaggcau                110


<210>  38

<211>  268
<212>  RNA
<213>  Artificial

<220>
<223>  RNA scaffold + aptamer

<400>  38
ugccuggcga ccauagcgau ugagacgguc ggguccaggu gcacaaaugu ggccuguuga      60

guagcgugug ggcucaauua gcgccgaugg uagugugggg uuuccccaug ugagaguagg      120

acaucgccag gcauugccug gcgaccauag cgauugagac ggucgggucc aggugcacaa      180

auguggccug uugaguagcg ugugggcuca auuagcgccg augguagugu gggguuuccc      240

caugugagag uaggacaucg ccaggcau                                          268


<210>  39
<211>  134
<212>  RNA
<213>  Artificial

<220>
<223>  RNA scaffold + aptamer

<400>  39
ugccuggcga ccauagcgau ugagacgguc ggguccaguc ccaacgaugu uggcuguuga      60

guagugugug ggcucaauua gcgccgaugg uagugugggg uuuccccaug ugagaguagg      120

acaucgccag gcau                                                          134


<210>  40
<211>  111
<212>  RNA
<213>  Artificial

<220>
<223>  RNA scaffold + aptamer

<400>  40
ugccuggcga ccauagcgau ugaaggacgg guccguugag uagaguguga gcaauuagcg      60

ccgaugguag ugugggguuu ccccauguga gaguaggaca ucgccaggca u               111


<210>  41
<211>  87
<212>  RNA
<213>  Artificial

<220>
<223>  RNA scaffold + aptamer


<220>
<221>  misc_feature
<222>  (68)..(71)
<223>  Insertion site for aptamer

<400>  41
uugucacgug uaugugggag guucgaagcu uuugcuugga cgaaccgccc acauacuuug      60

uugauccnnn nggaucaauc auggcaa                                          87



<210>  42
<211>  81
<212>  RNA
<213>  Artificial

<220>
<223>  RNA scaffold + aptamer



<220>
<221>  misc_feature
<222>  (62)..(65)
<223>  Insertion site for aptamer

<400>  42
uugucacgug uaugugggga agggacggug cggagaggag acccacauac uuuguugauc      60

cnnnnggauc aaucauggca a                                                81



<210>  43
<211>  83
<212>  RNA
<213>  Artificial

<220>
<223>  RNA scaffold + aptamer



<220>
<221>  misc_feature
<222>  (64)..(67)
<223>  Insertion site for aptamer

<400>  43
uugucacgug uaugugggcg agggaguggu gaggaugagg cgacccacau acuuuguuga      60

uccnnnngga ucaaucaugg caa                                              83



<210>  44
<211>  83
<212>  RNA
<213>  Artificial

<220>
<223>  RNA scaffold + aptamer



<220>
<221>  misc_feature
<222>  (64)..(67)
<223>  Insertion site for aptamer

<400>  44
uugucacgug uaugugggcg agggaguggu gaggaugagg cgacccacau acuuuguuga      60

uccnnnngga ucaaucaugg caa 83

<210> 45
<211> 83
<212> RNA
<213> Artificial

<220>
<223> RNA scaffold + aptamer


<220>
<221> misc_feature
<222> (64)..(67)
<223> Insertion site for aptamer

<400> 45
uugucacgug uaugugggcg agggaguggu gaggaugagg cgacccacau acuuuguuga 60

uccnnnngga ucaaucaugg caa 83


<210> 46
<211> 156
<212> RNA
<213> Artificial

<220>
<223> RNA scaffold + aptamer


<220>
<221> misc_feature
<222> (137)..(140)
<223> Insertion site for aptamer

<400> 46
uugucacgug uaugugggga cgcaacugaa ugaaauggug aaggacgggu ccaggugugg 60

cugcuucggc agugcagcuu guugaguaga gugugagcuc cguaacuagu cgcgucccca 120

cauacuuugu ugauccnnnn ggaucaauca uggcaa 156


<210> 47
<211> 153
<212> RNA
<213> Artificial

<220>
<223> RNA scaffold + aptamer


<220>
<221> misc_feature
<222> (134)..(137)
<223> Insertion site for aptamer

<400> 47
uugucacgug uaugugggga uguaacugaa ugaaauggug aaggacgggu ccaguaggcu 60

gcuucggcag ccuacuuguu gaguagagug ugagcuccgu aacuaguuac auccccacau 120

acuuuguuga uccnnnngga ucaaucaugg caa                                    153

<210>  48
<211>  105
<212>  RNA
<213>  Artificial

<220>
<223>  RNA scaffold + aptamer

<220>
<221>  misc_feature
<222>  (86)..(89)
<223>  Insertion site

<400>  48
uugucacgug uaugugggag acggucgggu ccagauauuc guaucugucg aguagagugu      60

gggcucccac auacuuuguu gauccnnnng gaucaaucau ggcaa                     105

<210>  49
<211>  86
<212>  RNA
<213>  Artificial

<220>
<223>  RNA scaffold + aptamer

<220>
<221>  misc_feature
<222>  (67)..(70)
<223>  Insertion site

<400>  49
uugucacgug uaugugggcg aggaaggagg ucugaggagg ucacugccca cauacuuugu      60

ugauccnnnn ggaucaauca uggcaa                                           86

<210>  50
<211>  110
<212>  RNA
<213>  Artificial

<220>
<223>  RNA scaffold + aptamer

<220>
<221>  misc_feature
<222>  (91)..(94)
<223>  Insertion site for aptamer

<400>  50
uugucacgug uaugugggag acggucgggu ccaggugcac aaauguggcc uguugaguag      60

cgugugggcu cccacauacu uuguugaucc nnnnggauca aucauggcaa                110

<210>  51
<211>  110
<212>  RNA
<213>  Artificial

<220>
<223>  RNA scaffold + aptamer


<220>
<221>  misc_feature
<222>  (91)..(94)
<223>  Insertion site for aptamer

<400>  51
uugucacgug uaugugggag acggucgggu ccagucccaa cgauguuggc uguugaguag      60

ugugugggcu cccacauacu uuguugaucc nnnnggauca aucauggcaa                110


<210>  52
<211>  87
<212>  RNA
<213>  Artificial

<220>
<223>  RNA scaffold + aptamer


<220>
<221>  misc_feature
<222>  (68)..(71)
<223>  Insertion site for aptamer

<400>  52
uugucacgug uaugugggaa ggacgggucc guugaguaga gugugagccc acauacuuug      60

uugauccnnn nggaucaauc auggcaa                                          87


<210>  53
<211>  87
<212>  RNA
<213>  Artificial

<220>
<223>  RNA scaffold + aptamer


<220>
<221>  misc_feature
<222>  (68)..(71)
<223>  Insertion site for aptatmer

<400>  53
uugccaugug uaugugggag guucgaagcu uuugcuugga cgaaccgccc acauacucug      60

augauccnnn nggaucauuc auggcaa                                          87


<210>  54
<211>  81

<212>  RNA
<213>  Artificial

<220>
<223>  RNA scaffold + aptamer


<220>
<221>  misc_feature
<222>  (62)..(65)
<223>  Insertion site for aptamer

<400>  54
uugccaugug uaugugggga agggacggug cggagaggag acccacauac ucugaugauc      60

cnnnnggauc auucauggca a                                                81


<210>  55
<211>  83
<212>  RNA
<213>  Artificial

<220>
<223>  RNA scaffold + aptamer


<220>
<221>  misc_feature
<222>  (64)..(67)
<223>  Insertion site for aptamer

<400>  55
uugccaugug uaugugggcg agggaguggu gaggaugagg cgacccacau acucugauga      60

uccnnnngga ucauucaugg caa                                              83


<210>  56
<211>  83
<212>  RNA
<213>  Artificial

<220>
<223>  RNA scaffold + aptamer


<220>
<221>  misc_feature
<222>  (64)..(67)
<223>  Insertion site for aptamer

<400>  56
uugccaugug uaugugggcg agggaguggu gaggaugagg cgacccacau acucugauga      60

uccnnnngga ucauucaugg caa                                              83


<210>  57
<211>  83
<212>  RNA
<213>  Artificial

<220>
<223>  RNA scaffold + aptamer


<220>
<221>  misc_feature
<223>  F30::Mango4

<220>
<221>  misc_feature
<222>  (64)..(67)
<223>  Insertion site for aptamer

<400>  57
uugccaugug uaugugggcg agggaguggu gaggaugagg cgacccacau acucugauga      60

uccnnnngga ucauucaugg caa                                              83


<210>  58
<211>  156
<212>  RNA
<213>  Artificial

<220>
<223>  RNA scaffold + aptamer


<220>
<221>  misc_feature
<222>  (137)..(140)
<223>  Insertion site for aptamer

<400>  58
uugccaugug uaugugggga cgcaacugaa ugaaauggug aaggacgggu ccaggugugg      60

cugcuucggc agugcagcuu guugaguaga gugugagcuc cguaacuagu cgcgucccca     120

cauacucuga ugauccnnnn ggaucauuca uggcaa                               156


<210>  59
<211>  153
<212>  RNA
<213>  Artificial

<220>
<223>  RNA scaffold + aptamer


<220>
<221>  misc_feature
<223>  F30::Spinach2

<220>
<221>  misc_feature
<222>  (134)..(137)
<223>  Insertion site for aptamer

<400>  59
uugccaugug uaugugggga uguaacugaa ugaaauggug aaggacgggu ccaguaggcu      60

gcuucggcag ccuacuuguu gaguagagug ugagcuccgu aacuaguuac auccccacau     120

acucugauga uccnnnngga ucauucaugg caa                                    153

<210>  60
<211>  105
<212>  RNA
<213>  Artificial

<220>
<223>  RNA scaffold + aptamer

<220>
<221>  misc_feature
<222>  (86)..(89)
<223>  Insertion site for aptamer

<400>  60
uugccaugug uaugugggag acggucgggu ccagauauuc guaucugucg aguagagugu      60

gggcucccac auacucugau gauccnnnng gaucauucau ggcaa                     105

<210>  61
<211>  86
<212>  RNA
<213>  Artificial

<220>
<223>  RNA scaffold + aptamer

<220>
<221>  misc_feature
<222>  (67)..(70)
<223>  Insertion site for aptamer

<400>  61
uugccaugug uaugugggcg aggaaggagg ucugaggagg ucacugccca cauacucuga      60

ugauccnnnn ggaucauuca uggcaa                                           86

<210>  62
<211>  110
<212>  RNA
<213>  Artificial

<220>
<223>  RNA scaffold + aptamer

<220>
<221>  misc_feature
<222>  (91)..(94)
<223>  Insertion site for aptamer

<400>  62
uugccaugug uaugugggag acggucgggu ccaggugcac aaauguggcc uguugaguag      60

cgugugggcu cccacauacu cugaugaucc nnnnggauca uucauggcaa                110

<210>  63
<211>  110
<212>  RNA
<213>  Artificial

<220>
<223>  RNA scaffold + aptamer


<220>
<221>  misc_feature
<222>  (91)..(94)
<223>  Insertion site for aptamer

<400>  63
uugccaugug uaugugggag acggucgggu ccagucccaa cgauguuggc uguugaguag      60

ugugugggcu cccacauacu cugaugaucc nnnnggauca uucauggcaa      110


<210>  64
<211>  87
<212>  RNA
<213>  Artificial

<220>
<223>  RNA scaffold + aptamer


<220>
<221>  misc_feature
<222>  (68)..(71)
<223>  Insertion site for aptamer

<400>  64
uugccaugug uaugugggaa ggacgggucc guugaguaga gugugagccc acauacucug      60

augauccnnn nggaucauuc auggcaa      87


<210>  65
<211>  52
<212>  RNA
<213>  Artificial

<220>
<223>  Aptamer

<400>  65
agacggucgg guccaggugc acaaaugugg ccuguugagu agcguguggg cu      52


<210>  66
<211>  52
<212>  RNA
<213>  Artificial

<220>
<223>  Aptamer

<400>  66
agacggucgg guccaguccc aacgauguug gcuguugagu aguguguggg cu      52

<210>  67
<211>  61
<212>  DNA
<213>  Artificial

<220>
<223>  Pf1 promoter

<400>  67
ctcgagcggg acggtcgaac cagcttcaag cgaccggatg agtatgttac agtagatagc      60

g                                                                      61


<210>  68
<211>  105
<212>  DNA
<213>  Artificial

<220>
<223>  Target RNA

<400>  68
agcgggagac cgctcgacct tagttctcct gttgcggggg agttcatggg atccaggtac      60

ccagggcgag gcttatccat tgcactccgg atgtgctgac ccctg                     105


<210>  69
<211>  105
<212>  DNA
<213>  Artificial

<220>
<223>  F30 with broccoli in insertion site 1 and TTCG in insertion site

<400>  69
ttgccatgtg tatgtgggag acggtcgggt ccagatattc gtatctgtcg agtagagtgt      60

gggctcccac atactctgat gatccttcgg gatcattcat ggcaa                     105


<210>  70
<211>  121
<212>  DNA
<213>  Artificial

<220>
<223>  TF1 Terminator sequence

<400>  70
gctagcatag cataaaataa cgccccacct tcttaacggg aggtggggcg ttatttttac      60

ggggatcgct tcttccgctt cctcgctcac tgactcgctg cgctcggtcg ttcggctgcg     120

g                                                                     121


<210>  71
<211>  414
<212>  DNA

<213>  Artificial

<220>
<223>  SEQ-ID No: 67-70 combined, with XbaI recognition site on 5'-end,
      SalI recognition site on 3'-end

<400>  71
ctgtctctag actcgagcgg gacggtcgaa ccagcttcaa gcgaccggat gagtatgtta      60

cagtagatag cgagcgggag accgctcgac cttagttctc ctgttgcggg ggagttcatg     120

ggatccaggt acccagggcg aggcttatcc attgcactcc ggatgtgctg acccctgttg     180

ccatgtgtat gtgggagacg gtcgggtcca gatattcgta tctgtcgagt agagtgtggg     240

ctcccacata ctctgatgat ccttcgggat cattcatggc aagctagcat agcataaaat     300

aacgccccac cttcttaacg ggaggtgggg cgttattttt acggggatcg cttcttccgc     360

ttcctcgctc actgactcgc tgcgctcggt cgttcggctg cgggtcgacc tgtc           414


<210>  72
<211>  309
<212>  DNA
<213>  Artificial

<220>
<223>  SEQ-ID No: 67, 68 and 70, with XbaI recognition site on 5'-end,
      SalI recognition site on 3'-end

<400>  72
ctgtctctag actcgagcgg gacggtcgaa ccagcttcaa gcgaccggat gagtatgtta      60

cagtagatag cgagcgggag accgctcgac cttagttctc ctgttgcggg ggagttcatg     120

ggatccaggt acccagggcg aggcttatcc attgcactcc ggatgtgctg acccctggct     180

agcatagcat aaaataacgc cccaccttct taacgggagg tggggcgtta tttttacggg     240

gatcgcttct tccgcttcct cgctcactga ctcgctgcgc tcggtcgttc ggctgcgggt     300

cgacctgtc                                                             309


<210>  73
<211>  20
<212>  DNA
<213>  Artificial

<220>
<223>  pJC1_check_f

<400>  73
tgaagaccgt caaccaaagg                                                  20


<210>  74
<211>  20
<212>  DNA
<213>  Artificial

<220>

<223>  pJC1_check_rev

<400>  74
tgccgggaag ctagagtaag                                                    20


<210>  75
<211>  6500
<212>  DNA
<213>  Artificial

<220>
<223>  pJC1-PF1-U1A-F30::broccoli/UUCG-TF1

<400>  75
ccgagaagtt ttttacaaaa ggcaaaaact ttttcgggat cgacagaaat aaaacgatcg        60

acggtacgca acaaaaaagc gtcaggatcg ccgtagagcg attgaagacc gtcaaccaaa       120

ggggaagcct ccaatcgacg cgacgcgcgc tctacggcga tcctgacgca gatttttagc       180

tatctgtcgc agcgccctca gggacaagcc acccgcacaa cgtcgcgagg gcgatcagcg       240

acgccgcagg gggatcctct agactcgagc gggacggtcg aaccagcttc aagcgaccgg       300

atgagtatgt tacagtagat agcgagcggg agaccgctcg accttagttc tcctgttgcg       360

ggggagttca tgggatccag gtacccaggg cgaggcttat ccattgcact ccggatgtgc       420

tgacccctgt tgccatgtgt atgtgggaga cggtcgggtc cagatattcg tatctgtcga       480

gtagagtgtg ggctcccaca tactctgatg atccttcggg atcattcatg gcaagctagc       540

atagcataaa ataacgcccc accttcttaa cgggaggtgg ggcgttattt ttacggggat       600

cgcttcttcc gcttcctcgc tcactgactc gctgcgctcg gtcgttcggc tgcgggtcga       660

cctgcagcaa tggcaacaac gttgcgcaaa ctattaactg gcgaactact tactctagct       720

tcccggcaac aattaataga ctggatggag gcggataaag ttgcaggacc acttctgcgc       780

tcggcccttc cggctggctg gtttattgct gataaatctg gagccggtga gcgtgggtct       840

cgcggtatca ttgcagcact ggggccagat ggtaagccct cccgtatcgt agttatctac       900

acgacgggga gtcaggcaac tatggatgaa cgaaatagac agatcgctga gataggtgcc       960

tcactgatta agcattggta actgtcagac caagtttact catatatact ttagattgat      1020

ttaaaacttc atttttaatt taaaaggatc taggtgaaga tcctttttga taatctcatg      1080

accaaaatcc cttaacgtga gttttcgttc cactgagcgt cagacccctt aataagatga      1140

tcttcttgag atcgttttgg tctgcgcgta atctcttgct ctgaaaacga aaaaaccgcc      1200

ttgcagggcg gtttttcgaa ggttctctga gctaccaact ctttgaaccg aggtaactgg      1260

cttggaggag cgcagtcacc aaaacttgtc ctttcagttt agccttaacc ggcgcatgac      1320

ttcaagacta actcctctaa atcaattacc agtggctgct gccagtggtg cttttgcatg      1380

tctttccggg ttggactcaa gacgatagtt accggataag gcgcagcggt cggactgaac      1440

ggggggttcg tgcatacagt ccagcttgga gcgaactgcc tacccggaac tgagtgtcag 1500

gcgtggaatg agacaaacgc ggccataaca gcggaatgac accggtaaac cgaaaggcag 1560

gaacaggaga gcgcacgagg gagccgccag ggggaaacgc ctggtatctt tatagtcctg 1620

tcgggtttcg ccaccactga tttgagcgtc agatttcgtg atgcttgtca ggggggcgga 1680

gcctatggaa aaacggcttt gccgcggccc tctcacttcc ctgttaagta tcttcctggc 1740

atcttccagg aaatctccgc cccgttcgta agccatttcc gctcgccgca gtcgaacgac 1800

cgagcgtagc gagtcagtga gcgaggaagc ggaatatatc ctgtatcaca tattctgctg 1860

acgcaccggt gcagcctttt ttctcctgcc acatgaagca cttcactgac accctcatca 1920

gtgccaacat agtaagccag tatacactcc gctagcgctg aggtctgcct cgtgaagaag 1980

gtgttgctga ctcataccag gcctgaatcg ccccatcatc cagccagaaa gtgagggagc 2040

cacggttgat gagagctttg ttgtaggtgg accagttggt gattttgaac ttttgctttg 2100

ccacggaacg gtctgcgttg tcgggaagat gcgtgatctg atccttcaac tcagcaaaag 2160

ttcgatttat tcaacaaagc cacgttgtgt ctcaaaatct ctgatgttac attgcacaag 2220

ataaaaatat atcatcatga acaataaaac tgtctgctta cataaacagt aatacaaggg 2280

gtgttatgag ccatattcaa cgggaaacgt cttgctcgag gccgcgatta aattccaaca 2340

tggatgctga tttatatggg tataaatggg ctcgcgataa tgtcgggcaa tcaggtgcga 2400

caatctatcg attgtatggg aagcccgatg cgccagagtt gtttctgaaa catggcaaag 2460

gtagcgttgc caatgatgtt acagatgaga tggtcagact aaactggctg acggaattta 2520

tgcctcttcc gaccatcaag cattttatcc gtactcctga tgatgcatgg ttactcacca 2580

ctgcgatccc cgggaaaaca gcattccagg tattagaaga atatcctgat tcaggtgaaa 2640

atattgttga tgcgctggca gtgttcctgc gccggttgca ttcgattcct gtttgtaatt 2700

gtccttttaa cagcgatcgc gtatttcgtc tcgctcaggc gcaatcacga atgaataacg 2760

gtttggttga tgcgagtgat tttgatgacg agcgtaatgg ctggcctgtt gaacaagtct 2820

ggaaagaaat gcataagctt ttgccattct caccggattc agtcgtcact catggtgatt 2880

tctcacttga taaccttatt tttgacgagg ggaaattaat aggttgtatt gatgttggac 2940

gagtcggaat cgcagaccga taccaggatc ttgccatcct atggaactgc ctcggtgagt 3000

tttctccttc attacagaaa cggctttttc aaaaatatgg tattgataat cctgatatga 3060

ataaattgca gtttcatttg atgctcgatg agtttttcta atcagaattg gttaattggt 3120

tgtaacactg gcagagcatt acgctgactt gacgggacgg cggctttgtt gaataaatcg 3180

aacttttgct gagttgaagg atcagatcac gcatcttccc gacaacgcag accgttccgt 3240

ggcaaagcaa aagttcaaaa tcaccaactg gtccacctac aacaaagctc tcatcaaccg 3300

tggctccctc actttctggc tggatgatgg ggcgattcag gcctggtatg agtcagcaac 3360

accttcttca cgaggcagac ctcagcgctc aaagatgcag gggtaaaagc taaccgcatc 3420

tttaccgaca aggcatccgg cagttcaaca gatcgggaag ggctggattt gctgaggatg 3480

aaggtggagg aaggtgatgt cattctggtg aagaagctcg accgtcttgg ccgcgacacc 3540

gccgacatga tccaactgat aaaagagttt gatgctcagg gtgtagcggt tcggtttatt 3600

gacgacggga tcagtaccga cggtgatatg gggcaaatgg tggtcaccat cctgtcggct 3660

gtggcacagg ctgaacgccg gaggatcaag tcggtcaagc caagcgcaac cagcggcacc 3720

gccgcgagca acgtcgcaag ggcgatcagg ggacgatttt tgcgaagaat ttccacggta 3780

agaatccaat ctctcgaatt tagggtgaaa gaagcttggc ataggggtgt gcacgaactc 3840

ggtggaggaa atttccgcgg ggcaaggctt cgcgaagcgg agtcgcggca gtggctttga 3900

agatctttgg gagcagtcct tgtgcgctta cgaggtgagc cggtggggaa ccgttatctg 3960

cctatggtgt gagcccccct agagagcttc aagagcaatc agcccgacct agaaaggagg 4020

ccaagagaga gacccctacg gggggaaccg ttttctgcct acgagatggc acatttactg 4080

ggaagcttta cggcgtcctc gtggaagttc aatgcccgca gacttaagtg ctctattcac 4140

ggtctgacgt gacacgctaa attcagacat agcttcattg attgtcggcc acgagccagt 4200

ctctccctca acagtcataa accaacctgc aatggtcaag cgatttcctt tagctttcct 4260

agcttgtcgt tgactggact tagctagttt ttctcgctgt gctcgggcgt actcactgtt 4320

tgggtctttc cagcgttctg cggccttttt accgccacgt cttcccatag tggccagagc 4380

ttttcgccct cggctgctct gcgtctctgt ctgacgagca gggacgactg gctggccttt 4440

agcgacgtag ccgcgcacac gtcgcgccat cgtctggcgg tcacgcatcg gcggcagatc 4500

aggctcacgg ccgtctgctc cgaccgcctg agcgacggtg taggcacgct cgtaggcgtc 4560

gatgatcttg gtgtctttta ggcgctcacc agccgctttt aactggtatc ccacagtcaa 4620

agcgtggcga aaagccgtct catcacgggc ggcacgccct ggagcagtcc agaggacacg 4680

gacgccgtcg atcagctctc cagacgcttc agcggcgctc ggcaggcttg cttcaagcgt 4740

ggcaagtgct tttgcttccg cagtggcttt tcttgccgct tcgatacgtg cccgtccgct 4800

agaaaactcc tgctcatagc gttttttagg tttttctgtg cctgagatca tgcgagcaac 4860

ctccataaga tcagctaggc gatccacgcg attgtgctgg gcatgccagc ggtacgcggt 4920

gggatcgtcg gagacgtgca gtggccaccg gctcagccta tgtgaaaaag cctggtcagc 4980

gccgaaaacg cgggtcattt cctcggtcgt tgcagccagc aggcgcatat tcgggctgct 5040

catgcctgct gcggcataca ccggatcaat gagccagatg agctggcatt tcccgctcag 5100

tggattcacg ccgatccaag ctggcgcttt ttccaggcgt gcccagcgct ccaaaatcgc 5160

gtagacctcg gggtttacgt gctcgatttt cccgccggcc tggtggctcg gcacatcaat 5220

gtccaggaca agcacggctg cgtgctgcgc gtgcgtcaga gcaacatact ggcaccgggc 5280

aagcgatttt gaaccaactc ggtataactt cggctgtgtt tctcccgtgt ccgggtcttt 5340

gatccaagcg ctggcgaagt cgcgggtctt gctgccctgg aaattttctc tgcccaggtg 5400

agcgaggaat tcgcggcggt cttcgctcgt ccagccacgt gatcgcagcg cgagctcggg 5460

atgggtgtcg aacagatcag cggaaaattt ccaggccggt gtgtcaatgt ctcgtgaatc 5520

cgctagagtc atttttgagc gctttctccc aggtttggac tgggggttag ccgacgccct 5580

gtgagttacc gctcacgggg cgttcaacat ttttcaggta ttcgtgcagc ttatcgcttc 5640

ttgccgcctg tgcgcttttt cgacgcgcga cgctgctgcc gattcggtgc aggtggtggc 5700

ggcgctgaca cgtcctgggc ggccacggcc acacgaaacg cggcatttac gatgtttgtc 5760

atgcctgcgg gcaccgcgcc acgatcgcgg ataattctcg ctgccgcttc cagctctgtg 5820

acgaccatgg ccaaaatttc gctcggggga cgcacttcca gcgccatttg cgacctagcc 5880

gcctccagct cctcggcgtg gcgtttgttg gcgcgctcgc ggctggctgc ggcacgacac 5940

gcatctgagc aatattttgc gcgccgtcct cgcgggtcag gccggggagg aatcaggcca 6000

ccgcagtagg cgcaactgat tcgatcctcc actactgtgc gtcctcctgg cgctgccgag 6060

cacgcagctc gtcagccagc tcctcaagat ccgccacgag agtttctagg tcgctcgcgg 6120

cactggccca gtctcgtgat gctggcgcgt ccgtcgtatc gagagctcgg aaaaatccga 6180

tcaccgtttt taaatcgacg gcagcatcga gcgcgtcgga ctccagcgcg acatcagaga 6240

gatccatagc tgatgattcg ggccaatttt ggtacttcgt cgtgaaggtc atgacaccat 6300

tataacgaac gttcgttaaa gtttttggcg gaaaatcacg cggcacgaaa attttcacga 6360

agcgggactt tgcgcagctc aggggtgcta aaaattttgt atcgcacttg atttttccga 6420

aagacagatt atctgcaaac ggtgtgtcgt atttctggct tggtttttaa aaaatctgga 6480

atcgaaaatt tgcggggcga 6500

<210> 76
<211> 6395
<212> DNA
<213> Artificial

<220>
<223> pJC1-PF1-U1A-TF1

<400> 76
ccgagaagtt ttttacaaaa ggcaaaaact ttttcgggat cgacagaaat aaaacgatcg 60

acggtacgca acaaaaaagc gtcaggatcg ccgtagagcg attgaagacc gtcaaccaaa 120

ggggaagcct ccaatcgacg cgacgcgcgc tctacggcga tcctgacgca gatttttagc 180

tatctgtcgc agcgccctca gggacaagcc acccgcacaa cgtcgcgagg gcgatcagcg 240

acgccgcagg gggatcctct agactcgagc gggacggtcg aaccagcttc aagcgaccgg 300

atgagtatgt tacagtagat agcgagcggg agaccgctcg accttagttc tcctgttgcg 360

ggggagttca tgggatccag gtacccaggg cgaggcttat ccattgcact ccggatgtgc 420

tgacccctgg ctagcatagc ataaaataac gccccacctt cttaacggga ggtggggcgt 480

tatttttacg gggatcgctt cttccgcttc ctcgctcact gactcgctgc gctcggtcgt 540

tcggctgcgg gtcgacctgc agcaatggca acaacgttgc gcaaactatt aactggcgaa 600

ctacttactc tagcttcccg gcaacaatta atagactgga tggaggcgga taaagttgca 660

ggaccacttc tgcgctcggc ccttccggct ggctggttta ttgctgataa atctggagcc 720

ggtgagcgtg ggtctcgcgg tatcattgca gcactggggc cagatggtaa gccctcccgt 780

atcgtagtta tctacacgac ggggagtcag gcaactatgg atgaacgaaa tagacagatc 840

gctgagatag gtgcctcact gattaagcat tggtaactgt cagaccaagt ttactcatat 900

atactttaga ttgatttaaa acttcatttt taatttaaaa ggatctaggt gaagatcctt 960

tttgataatc tcatgaccaa aatcccttaa cgtgagtttt cgttccactg agcgtcagac 1020

cccttaataa gatgatcttc ttgagatcgt tttggtctgc gcgtaatctc ttgctctgaa 1080

aacgaaaaaa ccgccttgca gggcggtttt tcgaaggttc tctgagctac caactctttg 1140

aaccgaggta actggcttgg aggagcgcag tcaccaaaac ttgtcctttc agtttagcct 1200

taaccggcgc atgacttcaa gactaactcc tctaaatcaa ttaccagtgg ctgctgccag 1260

tggtgctttt gcatgtcttt ccgggttgga ctcaagacga tagttaccgg ataaggcgca 1320

gcggtcggac tgaacggggg gttcgtgcat acagtccagc ttggagcgaa ctgcctaccc 1380

ggaactgagt gtcaggcgtg gaatgagaca aacgcggcca taacagcgga atgacaccgg 1440

taaaccgaaa ggcaggaaca ggagagcgca cgagggagcc gccaggggga aacgcctggt 1500

atctttatag tcctgtcggg tttcgccacc actgatttga gcgtcagatt tcgtgatgct 1560

tgtcaggggg gcggagccta tggaaaaacg gctttgccgc ggccctctca cttccctgtt 1620

aagtatcttc ctggcatctt ccaggaaatc tccgccccgt tcgtaagcca tttccgctcg 1680

ccgcagtcga acgaccgagc gtagcgagtc agtgagcgag gaagcggaat atatcctgta 1740

tcacatattc tgctgacgca ccggtgcagc cttttttctc ctgccacatg aagcacttca 1800

ctgacaccct catcagtgcc aacatagtaa gccagtatac actccgctag cgctgaggtc 1860

tgcctcgtga agaaggtgtt gctgactcat accaggcctg aatcgcccca tcatccagcc 1920

agaaagtgag ggagccacgg ttgatgagag ctttgttgta ggtggaccag ttggtgattt 1980

tgaacttttg ctttgccacg gaacggtctg cgttgtcggg aagatgcgtg atctgatcct 2040

tcaactcagc aaaagttcga tttattcaac aaagccacgt tgtgtctcaa aatctctgat 2100

gttacattgc acaagataaa aatatatcat catgaacaat aaaactgtct gcttacataa 2160

acagtaatac aaggggtgtt atgagccata ttcaacggga aacgtcttgc tcgaggccgc 2220

gattaaattc caacatggat gctgatttat atgggtataa atgggctcgc gataatgtcg 2280

ggcaatcagg tgcgacaatc tatcgattgt atgggaagcc cgatgcgcca gagttgtttc 2340

tgaaacatgg caaaggtagc gttgccaatg atgttacaga tgagatggtc agactaaact 2400

ggctgacgga atttatgcct cttccgacca tcaagcattt tatccgtact cctgatgatg 2460

catggttact caccactgcg atccccggga aaacagcatt ccaggtatta gaagaatatc 2520

ctgattcagg tgaaaatatt gttgatgcgc tggcagtgtt cctgcgccgg ttgcattcga 2580

ttcctgtttg taattgtcct tttaacagcg atcgcgtatt tcgtctcgct caggcgcaat 2640

cacgaatgaa taacggtttg gttgatgcga gtgattttga tgacgagcgt aatggctggc 2700

ctgttgaaca agtctggaaa gaaatgcata agcttttgcc attctcaccg gattcagtcg 2760

tcactcatgg tgatttctca cttgataacc ttatttttga cgaggggaaa ttaataggtt 2820

gtattgatgt tggacgagtc ggaatcgcag accgatacca ggatcttgcc atcctatgga 2880

actgcctcgg tgagttttct ccttcattac agaaacggct ttttcaaaaa tatggtattg 2940

ataatcctga tatgaataaa ttgcagtttc atttgatgct cgatgagttt ttctaatcag 3000

aattggttaa ttggttgtaa cactggcaga gcattacgct gacttgacgg gacggcggct 3060

ttgttgaata aatcgaactt ttgctgagtt gaaggatcag atcacgcatc ttcccgacaa 3120

cgcagaccgt tccgtggcaa agcaaaagtt caaaatcacc aactggtcca cctacaacaa 3180

agctctcatc aaccgtggct ccctcacttt ctggctggat gatggggcga ttcaggcctg 3240

gtatgagtca gcaacacctt cttcacgagg cagacctcag cgctcaaaga tgcaggggta 3300

aaagctaacc gcatctttac cgacaaggca tccggcagtt caacagatcg ggaagggctg 3360

gatttgctga ggatgaaggt ggaggaaggt gatgtcattc tggtgaagaa gctcgaccgt 3420

cttggccgcg acaccgccga catgatccaa ctgataaaag agtttgatgc tcagggtgta 3480

gcggttcggt ttattgacga cgggatcagt accgacggtg atatggggca aatggtggtc 3540

accatcctgt cggctgtggc acaggctgaa cgccggagga tcaagtcggt caagccaagc 3600

gcaaccagcg gcaccgccgc gagcaacgtc gcaagggcga tcaggggacg atttttgcga 3660

agaatttcca cggtaagaat ccaatctctc gaatttaggg tgaaagaagc ttggcatagg 3720

ggtgtgcacg aactcggtgg aggaaatttc cgcggggcaa ggcttcgcga agcggagtcg 3780

cggcagtggc tttgaagatc tttgggagca gtccttgtgc gcttacgagg tgagccggtg 3840

gggaaccgtt atctgcctat ggtgtgagcc cccctagaga gcttcaagag caatcagccc 3900

gacctagaaa ggaggccaag agagagaccc ctacgggggg aaccgttttc tgcctacgag 3960

atggcacatt tactgggaag ctttacggcg tcctcgtgga agttcaatgc ccgcagactt 4020

aagtgctcta ttcacggtct gacgtgacac gctaaattca gacatagctt cattgattgt 4080

cggccacgag ccagtctctc cctcaacagt cataaaccaa cctgcaatgg tcaagcgatt 4140

tcctttagct ttcctagctt gtcgttgact ggacttagct agtttttctc gctgtgctcg 4200

ggcgtactca ctgtttgggt ctttccagcg ttctgcggcc tttttaccgc cacgtcttcc 4260

catagtggcc agagcttttc gccctcggct gctctgcgtc tctgtctgac gagcagggac 4320

gactggctgg cctttagcga cgtagccgcg cacacgtcgc gccatcgtct ggcggtcacg 4380

catcggcggc agatcaggct cacggccgtc tgctccgacc gcctgagcga cggtgtaggc 4440

acgctcgtag gcgtcgatga tcttggtgtc ttttaggcgc tcaccagccg cttttaactg 4500

gtatcccaca gtcaaagcgt ggcgaaaagc cgtctcatca cgggcggcac gccctggagc 4560

agtccagagg acacggacgc cgtcgatcag ctctccagac gcttcagcgg cgctcggcag 4620

gcttgcttca agcgtggcaa gtgcttttgc ttccgcagtg gcttttcttg ccgcttcgat 4680

acgtgcccgt ccgctagaaa actcctgctc atagcgtttt ttaggttttt ctgtgcctga 4740

gatcatgcga gcaacctcca taagatcagc taggcgatcc acgcgattgt gctgggcatg 4800

ccagcggtac gcggtgggat cgtcggagac gtgcagtggc caccggctca gcctatgtga 4860

aaaagcctgg tcagcgccga aaacgcgggt catttcctcg gtcgttgcag ccagcaggcg 4920

catattcggg ctgctcatgc ctgctgcggc atacaccgga tcaatgagcc agatgagctg 4980

gcatttcccg ctcagtggat tcacgccgat ccaagctggc gctttttcca ggcgtgccca 5040

gcgctccaaa atcgcgtaga cctcggggtt tacgtgctcg attttcccgc cggcctggtg 5100

gctcggcaca tcaatgtcca ggacaagcac ggctgcgtgc tgcgcgtgcg tcagagcaac 5160

atactggcac cgggcaagcg attttgaacc aactcggtat aacttcggct gtgtttctcc 5220

cgtgtccggg tctttgatcc aagcgctggc gaagtcgcgg gtcttgctgc cctggaaatt 5280

ttctctgccc aggtgagcga ggaattcgcg gcggtcttcg ctcgtccagc cacgtgatcg 5340

cagcgcgagc tcgggatggg tgtcgaacag atcagcggaa aatttccagg ccggtgtgtc 5400

aatgtctcgt gaatccgcta gagtcatttt tgagcgcttt ctcccaggtt tggactgggg 5460

gttagccgac gccctgtgag ttaccgctca cggggcgttc aacatttttc aggtattcgt 5520

gcagcttatc gcttcttgcc gcctgtgcgc tttttcgacg cgcgacgctg ctgccgattc 5580

ggtgcaggtg gtggcggcgc tgacacgtcc tgggcggcca cggccacacg aaacgcggca 5640

tttacgatgt ttgtcatgcc tgcgggcacc gcgccacgat cgcggataat tctcgctgcc 5700

gcttccagct ctgtgacgac catggccaaa atttcgctcg ggggacgcac ttccagcgcc 5760

atttgcgacc tagccgcctc cagctcctcg gcgtggcgtt tgttggcgcg ctcgcggctg 5820

gctgcggcac gacacgcatc tgagcaatat tttgcgcgcc gtcctcgcgg gtcaggccgg 5880

ggaggaatca ggccaccgca gtaggcgcaa ctgattcgat cctccactac tgtgcgtcct 5940

cctggcgctg ccgagcacgc agctcgtcag ccagctcctc aagatccgcc acgagagttt 6000

ctaggtcgct cgcggcactg gcccagtctc gtgatgctgg cgcgtccgtc gtatcgagag 6060

ctcggaaaaa tccgatcacc gtttttaaat cgacggcagc atcgagcgcg tcggactcca 6120

gcgcgacatc agagagatcc atagctgatg attcgggcca attttggtac ttcgtcgtga 6180

aggtcatgac accattataa cgaacgttcg ttaaagtttt tggcggaaaa tcacgcggca 6240

cgaaaatttt cacgaagcgg gactttgcgc agctcagggg tgctaaaaat tttgtatcgc 6300

acttgatttt tccgaaagac agattatctg caaacggtgt gtcgtatttc tggcttggtt 6360

tttaaaaaat ctggaatcga aaatttgcgg ggcga 6395

<210> 77
<211> 148
<212> RNA
<213> Artificial

<220>
<223> Scaffold with two aptamers

<400> 77
   uugccaugug uaugugggag acggucgggu ccagauauuc guaucugucg aguagagugu 60
   gggcucccac auacucugau gauccagacg gucgggucca gauauucgua ucugucgagu 120
   agagaguggg cuggaucauu cauggcaa 148

<210> 78
<211> 124
<212> RNA
<213> Artificial

<220>
<223> Scaffold with two aptamers

<400> 78
uugccaugug uaugugggag acggucgggu ccagauauuc guaucugucg aguagagugu 60

gggcucccac auacucugau gauccgaagg gacggugcgg agaggagagg aucauucaug 120

gcaa 124

## Description

### Field of the invention

The invention pertains to methods for optimizing the expression of heterologous RNA in cells. Likewise, the invention pertains to cells that allow quantification of the expressed RNA.

### Technical background

Research in recent years has brought the awareness that RNA, far from being merely a transition molecule, fulfills a variety of functions, both regulatory and enzymatic. For example, it has been described that many small RNAs play key roles in the regulation of gene expression and that higher-order structures in RNA sequences, such as riboswitches or ribozymes, act as regulators of mRNA expression (Breaker, R. R. Natural and engineered nucleic acids as tools to explore biology. Nature 2004:432,838-845. Nellen, W., and C. Hammann. Small RNAs: analysis and regulatory functions. Nucleic acids and molecular biology. Springer-Verlag 2005, Heidelberg, Germany.). Based on their function as transition molecules (mRNA), as artificial small interfering RNA-molecules (siRNAs), as catalytically active RNA-molecules (ribozymes), as regulatory or interacting RNA-molecules (RNA-aptamers), RNA has versatile potential as active ingredient in therapeutics, biopesticides and other applications (Khan, A. U. Ribozyme: a clinical tool. Clin. Chim. Acta 2006:367,20-27. Fletcher, S. J., Reeves, P. T., Hoang, B. T. & Mitter, N. A Perspective on RNAi-Based Biopesticides. Front. Plant Sci. 11, (2020). Cagliari, D. et al. Management of Pest Insects and Plant Diseases by Non-Transformative RNAi. Frontiers in Plant Science vol. 10 (2019). Zotti, M. et al. RNA interference technology in crop protection against arthropod pests, pathogens and nematodes. Pest Management Science vol. 74 1239-1250 (2018). Vallazza, B. et al. Recombinant messenger RNA technology and its application in cancer immunotherapy, transcript replacement therapies, pluripotent stem cell induction, and beyond. Wiley Interdisciplinary Reviews: RNA vol. 6 471-499 (2015). Sahin, U., Karikó, K. & Türeci, O. mRNA-based therapeutics-developing a new class of drugs. Nature Reviews Drug Discovery vol. 13 759-780 (2014). Pardi, N., Hogan, M. J. & Weissman, D. Recent advances in mRNA vaccine technology. Current Opinion in Immunology vol. 65 14-20 (2020).). In order to perform the assays required for further elucidating these functions and in order to provide RNA-based active ingredients for application, there is a need for techniques that are capable of producing large quantities of a given RNA.

One of the most straightforward ways to generate RNA is RNA *in vitro* transcription. This approach is based on imitation of the enzymatic processes that govern RNA synthesis in all forms of life. A common system used for this purpose is the T7 RNA polymerase that, starting from a DNA template, can yield up to milligram quantities of RNA in a few hours of reaction time. However, not all DNA sequences are equally suitable for transcription via the T7 polymerase. In addition, the problem of unspecific addition of nucleotides to the 3' end results in inhomogeneity that can be crucial when examining regulatory functions. Besides, this method is also labor-intensive. Moreover, scalability of the *in vitro* transcription reaction and thus maximal achievable amounts of a given RNA are limited, making it challenging and costly to provide a given RNA in large quantities in reasonable time.

Chemical synthesis is currently commonly used for the production of RNA oligonucleotides of less than 10 nucleotides and up to 80 nucleotides. Synthesis is performed on solid supports such as polystyrene or controlled-pore glass and involves addition of the respective nucleotides in 3' to 5' direction. Because reaction conditions can be optimized, chemical synthesis allows the production of any given RNA irrespective of its sequence. However, the method is *per se* not suitable for producing larger RNA molecules having more than 100 nucleotides and is also costly.

Instead, large RNA molecules could be effectively produced by recombinant expression systems, similar to industrial production of recombinant proteins. *E. coli* has been used to this end, but not without difficulties: Degradation by intracellular RNases, large 3'-end and 5'-end heterogeneity of the transcripts and low RNA-titers have hampered extensive application (Ponchon L, Dardel F. Recombinant RNA technology: the tRNA scaffold. Nat Methods. 2007;4:571-6.). Alternative hosts may offer a better solution (Suzuki H, Ando T, Umekage S, Tanaka T, Kikuchi Y. Extracellular production of an RNA aptamer by ribonuclease-free marine bacteria harboring engineered plasmids: a proposal for industrial RNA drug production. Appl Environ Microbiol. 2010;76:786-93.). However, up to now, they have not been developed yet to a stage where they provide a viable alternative to existing systems (Baronti, L., Karlsson, H., Marušič, M. et al. A guide to large-scale RNA sample preparation. Anal Bioanal Chem. 2018:410, 3239-3252.).

### Objective technical problem

In order to optimize recombinant RNA expression systems, there is a need for a fast and reliable method for identifying cells that show a high expression of a given RNA molecule.

### Summary of the invention

The problem is solved by a method for optimizing the production of a RNA sequence of interest in a cell, comprising the steps of:
a) introducing into a plurality of cells a vector capable of expressing a heterologous RNA of interest, wherein said RNA is tagged with a RNA tag comprising
   - an aptamer capable of stabilizing a fluorophore and
   - a scaffold capable of stabilizing the aptamer;
b) culturing the cells in a culture medium under conditions that allow expression of the heterologous RNA of interest;
c) adding said fluorophore to the culture medium;
d) identifying those cells that show the highest intensity of fluorescence and therefore a high expression of the RNA of interest.

Likewise, the problem is solved by a method for producing a RNA of interest, comprising the steps of
a) introducing into a plurality of cells a vector capable of expressing a heterologous RNA of interest, wherein said RNA is tagged with a RNA tag comprising an aptamer capable of stabilizing a fluorophore and a scaffold capable of stabilizing the aptamer;
b) culturing the cells in a culture medium under conditions that allow expression of the heterologous RNA of interest;
c) adding said fluorophore to the culture medium;
d) identifying and isolating those cells that show the highest intensity of fluorescence and therefore a high expression of the RNA of interest;
e) removing the first vector of step a) from the cells isolated in step d);
f) introducing a second vector capable of expressing the heterologous RNA of interest without the RNA tag into the cells obtained in step e);
g) producing the RNA of interest by culturing the cells obtained in step f).

Furthermore, the problem is solved by a microbial cell harboring a vector capable of expressing a heterologous RNA of interest, wherein said RNA is tagged with a RNA tag comprising
- an aptamer capable of stabilizing a fluorophore and
- an RNA scaffold capable of stabilizing the aptamer.

### Brief description of the figures

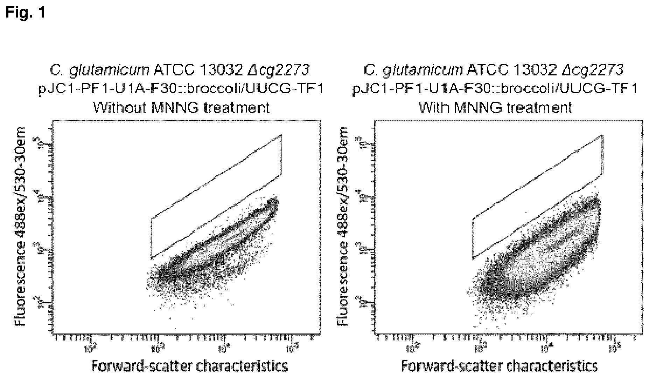

Fig. 1 shows the cytometric acquisition of the forward-scatter characteristics (x-axis) and 530 nm fluorescence characteristics (y-axis) of 100,000 representative cells of *C. glutamicum* ATCC 13032 *Δcg2273* pJC1-PF1-U1A-F30::broccoli/UUCG-TF1 without (left panel) and with (right panel) N-Methyl-N'-nitro-N-nitrosoguanidine (MNNG) treatment. The rectangle represents the sorting gate used for the isolation of highly fluorescent cells. (see Example 1d).

### Detailed description

As used herein, the terms "comprise" and "comprising" are understood to mean both "contain/containing" and "consist/consisting".

In one aspect, the invention is directed to a method for optimizing the production of a RNA sequence of interest in a cell, comprising the steps of:
a) introducing into a plurality of cells a vector capable of expressing a heterologous RNA of interest, wherein said RNA is tagged with a RNA tag comprising
   - an aptamer capable of stabilizing a fluorophore and
   - a scaffold capable of stabilizing the aptamer;
b) culturing the cells in a culture medium under conditions that allow expression of the heterologous RNA of interest;
c) adding said fluorophore to the culture medium;
d) identifying those cells that show the highest intensity of fluorescence and therefore a high expression of the RNA of interest.

In the context of the invention, a cell can be a eukaryotic or a prokaryotic cell. In one embodiment, the cell is a microbial cell. Microbial cells are useful for producing molecules such as DNA, RNA or proteins. They can be differentiated into Gram positive and Gram negative microbes. Preferably, the microbial cells according to the invention are Gram positive microbial cells. In one aspect of the invention, the microbial cells according to the invention are from the genus of *Corynebacterium,* in particular *Corynebacterium glutamicum.* The cells according to and used in the invention are herein also referred to as host cells.

First, a vector capable of expressing a heterologous RNA of interest is introduced into the cells. The vector can be any vector suitable for expressing a RNA in a host cell, such as a plasmid; a viral vector, e.g. a retrovirus, lentivirus, adenovirus, adeno-associated virus or Lambda phage; or an artificial chromosome, e.g. a BAC, YAC or HAC.

The vector used in the invention is capable of expressing a RNA of interest. The expression of the RNA of interest can be constitutive or conditional. For example, expression of the RNA of interest may be induced by addition of acetate, anhydrotetracycline, arabinose, gluconate, isopropyl β-D-1-thiogalactopyranoside (IPTG), light, maltose, methanol, propionate or by increasing the cultivation temperature.

Other than the elements listed below, the vector may comprise additional elements that are necessary for or enhance expression, molecular cloning and replication. For example, the vector may comprise selection or marker genes such as /*acZ* encoding beta-galactosidase, *luc* encoding luciferase, *cat* encoding chloramphenicol transferase or other resistance genes conveying resistance to antibiotics. The vector may also comprise an origin of replication, a multiple cloning site and/or transcriptional terminators.

The RNA of interest that is expressed by the vector is a heterologous RNA, i.e. an RNA molecule that is not expressed in the wildtype of the host cell. The sequence of the RNA of interest is not limited and can be any naturally or artificially occurring RNA sequence. In one embodiment, the RNA of interest is a mRNA, viral RNA, retroviral RNA, antisense RNA, replicon RNA, bicistronic or multicistronic RNA, small interfering RNA or immunostimulating RNA. In one embodiment, the RNA of interest has a length of 20 - 10000 nucleotides.

The RNA of interest is tagged with an RNA tag. "Tagged" herein means that the RNA tag is linked to the RNA of interest and that the tag is expressed together with the RNA of interest. Upon expression of the heterologous RNA from the vector, the RNA tag is not removed from the RNA of interest. According to the invention, it is not necessary that the tag is directly attached to the RNA of interest, although this is a preferred embodiment. But the RNA tag and the RNA of interest may also be separated by a nucleotide spacer sequence.

The RNA tag used in the invention comprises an aptamer. An "aptamer" is herein understood to refer to a RNA oligonucleotide that is capable of binding a small molecule fluorophore. Usually, aptamers are 10-100 base nucleic acid oligonucleotides that bind with high affinity to small molecules to induce their fluorescence. Before a binding event occurs, neither the aptamer nor the small molecule are strongly fluorescent but the binding of an aptamer to its target molecule activates the fluorescence of the target molecule. Aptamers capable of binding a molecule of interest can be generated via systematic evolution of ligands by exponential enrichment (SELEX) (Paige, J. S., Wu, K. Y., and Jaffrey, S. R. RNA mimics of green fluorescent protein. Science;2011;333, 642-6.). A variety of aptamers have been described in literature (Ouellet, J. (2016) RNA fluorescence with light-Up aptamers. Front. Chem. 4, 1-12. Bouhedda, F., Autour, A., and Ryckelynck, M. (2017) Light-Up RNA Aptamers and Their Cognate Fluorogens: From Their Development to Their Applications. Int. J. Mol. Sci. 19, 44.) . Preferred aptamer sequences to be used in the invention include:
**SEQ-ID No: 1**
   Aptamer II-mini3-4: AGGUUCGAAGCUUUUGCUUGGACGAACCG
**SEQ-ID No: 2**
   Mango: GAAGGGACGGUGCGGAGAGGAGA
**SEQ-ID No: 3**
   Mango2: CGAGGGAGUGGUGAGGAUGAGGCGA
**SEQ-ID No: 4**
   Mango3: CGAGGGAGUGGUGAGGAUGAGGCGA
**SEQ-ID No:** 5
   Mango4: CGAGGGAGUGGUGAGGAUGAGGCGA
**SEQ-ID No: 6**
   Spinach: GACGCAACUGAAUGAAAUGGUGAAGGACGGGUCCAGGUGUGGCUGCUUC GGCAGUGCAGCUUGUUGAGUAGAGUGUGAGCUCCGUAACUAGUCGCGUC
**SEQ-ID No: 7**
   Spinach2: GAUGUAACUGAAUGAAAUGGUGAAGGACGGGUCCAGUAGGCUGCUUCG GCAGCCUACUUGUUGAGUAGAGUGUGAGCUCCGUAACUAGUUACAUC
**SEQ-ID No: 8**
   Broccoli: AGACGGUCGGGUCCAGAUAUUCGUAUCUGUCGAGUAGAGUGUGGGCU
**SEQ-ID No: 13**
   Corn: CGAGGAAGGAGGUCUGAGGAGGUCACUG
**SEQ-ID No: 14**
   Orange broccoli: 
GAGACGCAACUGAAUGAAAUGGUGAAGGAGACGGUCGGGUCCAGGUGCACAAAUG
UGGCCUGUUGAGUAGCGUGUGGGCUCCGUAACUAGUCGCGUCAC
**SEQ-ID No: 65**
   Orange broccoli short version:
   AGACGGUCGGGUCCAGGUGCACAAAUGUGGCCUGUUGAGUAGCGUGUGGGCU
**SEQ-ID No: 15**
   Red broccoli: 
GAGACGCAACUGAAUGAAAUGUUUCGGAGACGGUCGGGUCCAGUCCCAACGAUG
UUGGCUGUUGAGUAGUGUGUGGGCUCCGUAACUAGUCGCGUCAC
**SEQ-ID No: 66**
   Red broccoli short version:
   AGACGGUCGGGUCCAGUCCCAACGAUGUUGGCUGUUGAGUAGUGUGUGGGCU
**SEQ-ID No: 16**
   Baby spinach: AAGGACGGGUCCGUUGAGUAGAGUGUGAG

The aptamers contained within the RNA tag used in the invention are capable of binding a fluorophore, i.e. an organic molecule that emits fluorescence upon light excitation. Light emission intensity essentially depends on binding of the fluorophore to the aptamer because the fluorophore's structure is stabilized when bound to the aptamer. This stabilization results in a preferred dissociation of excitation energy as fluorescence. That means that according to the invention, the fluorophores light emission strongly increases after binding to an aptamer.

The fluorophores used in the invention are small, non-toxic molecules that can easily enter into a cell. Pairs of aptamers and fluorophores that can bind to these aptamers have been previously described. Fluorophores that can be bound by aptamers include (Z)-4-(2-hydroxybenzylidene)-1,2-dimethyl-1H-imidazol-5(4H)-one (2-HBI), (5*Z*)-5-[(3,5-Difluoro-4-hydroxyphenyl)methylene]-3,5-dihydro-2,3-dimethyl-4H-imidazol-4-one (DFHBI), (5*Z*)-5-[(3,5-Difluoro-4-hydroxyphenyl)methylene]-3,5-dihydro-2-methyl-3-(2,2,2-trifluoroethyl)-4*H*-imidazol-4-one (DFHBI-1T), DFHBI-2T, 2-(4-(dimethylamino)benzylidene)-1H-indene-1,3(2H)-dione (DMABI), (Z)-4-(3,5- dimethyl-4-hydroxybenzylidene)-1,2-dimethyl-1H-imidazol- 5(4H)-one (DMHBI), 3,5-difluoro-4-hydroxybenzylidene imidazolinone-2-oxime (DFHO), 2-(2-Methylbenzo[d]thiazol-3-ium-3-yl)acetate (TO1), 4-[(E)-2-(acetylphenylamino)ethenyl]-1-methylquinolinium iodide (TO3) and (N-(6-aminohexyl)-2-(2,6-di-tert-butyl-4-(5-(4-methylpiperazin-1-yl)- 1H,1'H-2,5'-bibenzo[d]imidazol-2'-yl)phenoxy)acetamide) (Hoechst 1C).

In a preferred embodiment, the aptamer comprises SEQ-ID No: 1 and the fluorophore is Hoechst 1C. In another preferred embodiment, the aptamer comprises SEQ-ID No: 2 and the fluorophore is TO1 or TO3. In another preferred embodiment, the aptamer comprises SEQ-ID No: 3 and the fluorophore is TO1 or TO3. In another preferred embodiment, the aptamer comprises SEQ-ID No: 4 and the fluorophore is TO1 or TO3. In another preferred embodiment, the aptamer comprises SEQ-ID No: 5 and the fluorophore is TO1 or TO3. In another preferred embodiment, the aptamer comprises SEQ-ID No: 6 and the fluorophore is any of 2-HBI, DFHBI, DFHBI-1T, DFHBI-2T, DMABI or DMHBI. In another preferred embodiment, the aptamer comprises SEQ-ID No: 7 and the fluorophore is any of 2-HBI, DFHBI, DFHBI-1T, DFHBI-2T, DMABI or DMHBI. In another preferred embodiment, the aptamer comprises SEQ-ID No: 8 and the fluorophore is any of 2-HBI, DFHBI, DFHBI-1T, DFHBI-2T, DMABI or DMHBI. In another preferred embodiment, the aptamer comprises SEQ ID No: 13, 14, 15, 65 or 66 and the fluorophore is DFHO. In another preferred embodiment, the aptamer comprises SEQ ID No: 16 and the fluorophore is DHFBI or DHFBI-1T.

The RNA tag used in the invention further comprises an RNA scaffold. The RNA scaffold can be any nucleotide sequence that is capable of stabilizing the aptamer, supporting the formation of the functional aptamer structure and reducing aptamer degradation. A RNA scaffold according to the invention comprises at least one insertion site represented by NNNN. In the vectors used in the invention, NNNN is replaced by the respective aptamer site.

In one embodiment, the scaffold comprises two insertion sites, e.g. as in SEQ-ID No: 11 and 12. According to the invention, one or two aptamers may be inserted into such a scaffold. If two aptamers are inserted, the same or different aptamers may be inserted into the two insertion sites. Examples for this are SEQ-ID No: 77 and 78. If the same aptamer is inserted into both insertion sites, the fluorescence signal emitted after addition of the fluorophore will be stronger than if only one aptamer is present. Using two different aptamers has the advantage that cells expressing the RNA of interest will emit two different fluorescence signals. This may be beneficial to exclude false positives.

If only one aptamer is inserted into scaffolds having two insertion sites, the second insertion site may be replaced with any spacer sequence. In a preferred embodiment, the spacer sequences comprises four nucleotides. In a particularly preferred embodiment, the spacer sequence is *UUCG* or TTCG.

In a preferred embodiment, the RNA scaffold comprises one of the following sequences:
**SEQ-ID No: 9**
   tRNA^{Lys}: 
GCCCGGAUAGCUCAGUCGGUAGAGCAGNNNNCGGGUCCAGGGUUCAAGUCCCUG
UUCGGGCGCCA
**SEQ-ID No: 10**
   V5: 
UGCCUGGCGACCAUAGCGAUUGNNNCAAUUAGCGCCGAUGGUAGUGUGGGGUU
UCCCCAUGUGAGAGUAGGACAUCGCCAGGCAU
**SEQ-ID No: 11**
   F29: 
UUGUCACGUGUAUGUGGGNNNNCCCACAUACUUUGUUGAUCCNNNNGGAUCAAUC
AUGGCAA
**SEQ-ID No: 12**
   F30:

UUGCCAUGUGUAUGUGGGNNNNCCCACAUACUCUGAUGAUCCNNNNGGAUCAUUC
AUGGCAA

In one embodiment, the RNA tag comprises any of SEQ-ID No: 17 to 64, 69, 77 or 78. In a preferred embodiment, the RNA tag comprises SEQ-ID No: 69.
**SEQ-ID No: 17**
   tRNALys::Aptamer II-mini3-4 
GCCCGGAUAGCUCAGUCGGUAGAGCAGAGGUUCGAAGCUUUUGCUUGGACGAAC
CGCGGGUCCAGGGUUCAAGUCCCUGUUCGGGCGCCA
**SEQ-ID No: 18**
   tRNALys::Mango 
GCCCGGAUAGCUCAGUCGGUAGAGCAGGAAGGACGGUGCGGAGAGGAGACGGG
UCCAGGGUUCAAGUCCCUGUUCGGGCGCCA
**SEQ-ID No: 19**
   tRNALys::Mango2 
GCCCGGAUAGCUCAGUCGGUAGAGCAGCGAGGGAGUGGUGAGGAUGAGGCGACG
GGUCCAGGGUUCAAGUCCCUGUUCGGGCGCCA
**SEQ-ID No: 20**
   tRNALys::Mango3 
GCCCGGAUAGCUCAGUCGGUAGAGCAGCGAGGGAGUGGUGAGGAUGAGGCGACG
GGUCCAGGGUUCAAGUCCCUGUUCGGGCGCCA
**SEQ-ID No: 21** 
tRNALys::Mango4GCCCGGAUAGCUCAGUCGGUAGAGCAGCGAGGGAGUGGUGAGG
AUGAGGCGACGGGUCCAGGGUUCAAGUCCCUGUUCGGGCGCCA
**SEQ-ID No: 22**
   tRNALys::Spinach 
GCCCGGAUAGCUCAGUCGGUAGAGCAGGACGCAACUGAAUGAAAUGGUGAAGGAC
GGGUCCAGGUGUGGCUGCUUCGGCAGUGCAGCUUGUUGAGUAGAGUGUGAGCUC
CGUAACUAGUCGCGUCCGGGUCCAGGGUUCAAGUCCCUGUUCGGGCGCCA
**SEQ-ID No: 23**
   tRNALys::Spinach2 
GCCCGGAUAGCUCAGUCGGUAGAGCAGGAUGUAACUGAAUGAAAUGGUGAAGGAC
GGGUCCAGUAGGCUGCUUCGGCAGCCUACUUGUUGAGUAGAGUGUGAGCUCCGU
AACUAGUUACAUCCGGGUCCAGGGUUCAAGUCCCUGUUCGGGCGCCA
**SEQ-ID No: 24**
   tRNALys::Broccoli 
GCCCGGAUAGCUCAGUCGGUAGAGCAGAGACGGUCGGGUCCAGAUAUUCGUAUC
UGUCGAGUAGAGUGUGGGCUCGGGUCCAGGGUUCAAGUCCCUGUUCGGGCGCCA
**SEQ-ID No: 25**
   tRNALys::Corn 
GCCCGGAUAGCUCAGUCGGUAGAGCAGCGAGGAAGGAGGUCUGAGGAGGUCACU
GCGGGUCCAGGGUUCAAGUCCCUGUUCGGGCGCCA
**SEQ-ID No: 26**
   tRNALys::Orange broccoli 
GCCCGGAUAGCUCAGUCGGUAGAGCAGAGACGGUCGGGUCCAGGUGCACAAAUG
UGGCCUGUUGAGUAGCGUGUGGGCUCGGGUCCAGGGUUCAAGUCCCUGUUCGG
GCGCCA
**SEQ-ID No: 27**
   tRNALys::Red broccoli 
GCCCGGAUAGCUCAGUCGGUAGAGCAGAGACGGUCGGGUCCAGUCCCAACGAUG
UUGGCUGUUGAGUAGUGUGUGGGCUCGGGUCCAGGGUUCAAGUCCCUGUUCGG
GCGCCA
**SEQ-ID No: 28**
   tRNALys::Baby spinach 
GCCCGGAUAGCUCAGUCGGUAGAGCAGAAGGACGGUCCGUUGAGUAGAGUGUG
AGCGGGUCCAGGGUUCAAGUCCCUGUUCGGGCGCCA
**SEQ-ID No: 29**
   V5::Aptamer II-mini3-4 
UGCCUGGCGACCAUAGCGAUUGAGGUUCGAAGCUUUUGCUUGGACGAACCGCAA
UUAGCGCCGAUGGUAGUGUGGGGUUUCCCCAUGUGAGAGUAGGACAUCGCCAGG
CAU
**SEQ-ID No: 30**
   V5::Mango 
UGCCUGGCGACCAUAGCGAUUGGAAGGGACGGUGCGGAGAGGAGACAAUUAGCG
CCGAUGGUAGUGUGGGGUUUCCCCAUGUGAGAGUAGGACAUCGCCAGGCAU
**SEQ-ID No: 31**
   V5::Mango2 
UGCCUGGCGACCAUAGCGAUUGCGAGGGAGUGGUGAGGAUGAGGCGACAAUUAG
CGCCGAUGGUAGUGUGGGGUUUCCCCAUGUGAGAGUAGGACAUCGCCAGGCAU
**SEQ-ID No: 32**
   V5::Mango3 
UGCCUGGCGACCAUAGCGAUUGCGAGGGAGUGGUGAGGAUGAGGCGACAAUUAG
CGCCGAUGGUAGUGUGGGGUUUCCCCAUGUGAGAGUAGGACAUCGCCAGGCAU
**SEQ-ID No: 33**
   V5::Mango4 
UGCCUGGCGACCAUAGCGAUUGCGAGGGAGUGGUGAGGAUGAGGCGACAAUUAG
CGCCGAUGGUAGUGUGGGGUUUCCCCAUGUGAGAGUAGGACAUCGCCAGGCAU
**SEQ-ID No: 34**
   V5::Spinach 
UGCCUGGCGACCAUAGCGAUUGGACGCAACUGAAUGAAAUGGUGAAGGACGGGU
CCAGGUGUGGCUGCUUCGGCAGUGCAGCUUGUUGAGUAGAGUGUGAGCUCCGUA
ACUAGUCGCGUCCAAUUAGCGCCGAUGGUAGUGUGGGGUUUCCCCAUGUGAGAG
UAGGACAUCGCCAGGCAU
**SEQ-ID No:35**
   V5::Spinach2 
UGCCUGGCGACCAUAGCGAUUGGAUGUAACUGAAUGAAAUGGUGAAGGACGGGU
CCAGUAGGCUGCUUCGGCAGCCUACUUGUUGAGUAGAGUGUGAGCUCCGUAACU
AGUUACAUCCAAUUAGCGCCGAUGGUAGUGUGGGGUUUCCCCAUGUGAGAGUAG
GACAUCGCCAGGCAU
**SEQ-ID No: 36**
   V5::Broccoli 
UGCCUGGCGACCAUAGCGAUUGAGACGGUCGGGUCCAGAUAUUCGUAUCUGUCG
AGUAGAGUGUGGGCUCAAUUAGCGCCGAUGGUAGUGUGGGGUUUCCCCAUGUGA
GAGUAGGACAUCGCCAGGCAU
**SEQ-ID No: 37**
   V5::Corn 
UGCCUGGCGACCAUAGCGAUUGCGAGGAAGGAGGUCUGAGGAGGUCACUGCAAU
UAGCGCCGAUGGUAGUGUGGGGUUUCCCCAUGUGAGAGUAGGACAUCGCCAGGC
AU
SEQ-ID No: 38
   V5::Orange broccoli 
UGCCUGGCGACCAUAGCGAUUGAGACGGUCGGGUCCAGGUGCACAAAUGUGGCC
UGUUGAGUAGCGUGUGGGCUCAAUUAGCGCCGAUGGUAGUGUGGGGUUCCCCA
UGUGAGAGUAGGACAUCGCCAGGCAU
**SEQ-ID No: 39**
   V5::Red broccoli 
UGCCUGGCGACCAUAGCGAUUGAGACGGUCGGGUCCAGUCCCAACGAUGUUGGC
UGUUGAGUAGUGUGUGGGCUCAAUUAGCGCCGAUGGUAGUGUGGGGUUUCCCCA
UGUGAGAGUAGGACAUCGCCAGGCAU
**SEQ-ID No: 40**
   V5::Baby spinach 
UGCCUGGCGACCAUAGCGAUUGAAGGACGGGUCCGUUGAGUAGAGUGUGAGCAA
UUAGCGCCGAUGGUAGUGUGGGGUUUCCCCAUGUGAGAGUAGGACAUCGCCAGG
CAU
**SEQ-ID No: 41**
   F29::Aptamer II-mini3-4 
UUGUCACGUGUAUGUGGGAGGUUCGAAGCUUUUGCUUGGACGAACCGCCCACAU
ACUUUGUUGAUCCNNNNGGAUCAAUCAUGGCAA
**SEQ-ID No: 42**
   F29::Mango 
UUGUCACGUGUAUGUGGGGAAGGACGGUGCGGAGAGGAGACCCACAUACUUUG
UUGAUCCNNNNGGAUCAAUCAUGGCAA
**SEQ-ID No: 43**
   F29::Mango2 
UUGUCACGUGUAUGUGGGCGAGGGAGUGGUGAGGAUGAGGCGACCCACAUACUU
UGUUGAUCCNNNNGGAUCAAUCAUGGCAA
**SEQ-ID No: 44**
   F29::Mango3 
UUGUCACGUGUAUGUGGGCGAGGGAGUGGUGAGGAUGAGGCGACCCACAUACUU
UGUUGAUCCNNNNGGAUCAAUCAUGGCAA
**SEQ-ID No: 45**
   F29::Mango4 
UUGUCACGUGUAUGUGGGCGAGGGAGUGGUGAGGAUGAGGCGACCCACAUACUU
UGUUGAUCCNNNNGGAUCAAUCAUGGCAA
**SEQ-ID No: 46**
   F29::Spinach 
UUGUCACGUGUAUGUGGGGACGCAACUGAAUGAAAUGGUGAAGGACGGGUCCAG
GUGUGGCUGCUUCGGCAGUGCAGCUUGUUGAGUAGAGUGUGAGCUCCGUAACUA
GUCGCGUCCCCACAUACUUUGUUGAUCCNNNNGGAUCAAUCAUGGCAA
**SEQ-ID No: 47**
   F29::Spinach2 
UUGUCACGUGUAUGUGGGAUGUAACUGAAUGAAAUGGUGAAGGACGGUCCAG
UAGGCUGCUUCGGCAGCCUACUUGUUGAGUAGAGUGUGAGCUCCGUAACUAGUU
ACAUCCCCACAUACUUUGUUGAUCCNNNNGGAUCAAUCAUGGCAA
**SEQ-ID No: 48**
   F29::Broccoli 
UUGUCACGUGUAUGUGGGAGACGGUCGGGUCCAGAUAUUCGUAUCUGUCGAGUA
GAGUGUGGGCUCCCACAUACUUUGUUGAUCCNNNNGGAUCAAUCAUGGCAA
**SEQ-ID No: 49**
   F29::Corn 
UUGUCACGUGUAUGUGGGCGAGGAAGGAGGUCUGAGGAGGUCACUGCCCACAUA
CUUUGUUGAUCCNNNNGGAUCAAUCAUGGCAA
**SEQ-ID No: 50**
   F29::Orange broccoli 
UUGUCACGUGUAUGUGGGAGACGGUCGGGUCCAGGUGCACAAAUGUGGCCUGUU
GAGUAGCGUGUGGGCUCCCACAUACUUUGUUGAUCCNNNNGGAUCAAUCAUGGCA
A
**SEQ-ID No: 51**
   F29::Red broccoli UUGUCACGUGUAUGUGGGAGACGGUCGGGUCCAGUCCCAACGAUGUUGGCUGUU 
GAGUAGUGUGUGGGCUCCCACAUACUUUGUUGAUCCNNNNGGAUCAAUCAUGGCA
A
**SEQ-ID No: 52**
   F29::Baby spinach 
UUGUCACGUGUAUGUGGGAAGGACGGGUCCGUUGAGUAGAGUGUGAGCCCACAU
ACUUUGUUGAUCCNNNNGGAUCAAUCAUGGCAA
**SEQ-ID No: 53**
   F30::AptamerII-mini3-4 
UUGCCAUGUGUAUGUGGGAGGUUCGAAGCUUUUGCUUGGACGAACCGCCCACAU
ACUCUGAUGAUCCNNNNGGAUCAUUCAUGGCAA
**SEQ-ID No: 54**
   F30::Mango: 
UUGCCAUGUGUAUGUGGGAAGGACGGUGCGGAGAGGAGACCCACAUACUCUG
AUGAUCCNNNNGGAUCAUUCAUGGCAA
**SEQ-ID No: 55**
   F30::Mango2: 
UUGCCAUGUGUAUGUGGGCGAGGGAGUGGUGAGGAUGAGGCGACCCACAUACUC
UGAUGAUCCNNNNGGAUCAUUCAUGGCAA
**SEQ-ID No: 56**
   F30::Mango3: 
UUGCCAUGUGUAUGUGGGCGAGGGAGUGGUGAGGAUGAGGCGACCCACAUACUC
UGAUGAUCCNNNNGGAUCAUUCAUGGCAA
**SEQ-ID No: 57**
   F30::Mango4 
UUGCCAUGUGUAUGUGGGCGAGGGAGUGGUGAGGAUGAGGCGACCCACAUACUC
UGAUGAUCCNNNNGGAUCAUUCAUGGCAA
**SEQ-ID No: 58**
   F30::Spinach UUGCCAUGUGUAUGUGGGGACGCAACUGAAUGAAAUGGUGAAGGACGGGUCCAG 
GUGUGGCUGCUUCGGCAGUGCAGCUUGUUGAGUAGAGUGUGAGCUCCGUAACUA
GUCGCGUCCCCACAUACUCUGAUGAUCCNNNNGGAUCAUUCAUGGCAA
**SEQ-ID No: 59**
   F30::Spinach2 
UUGCCAUGUGUAUGUGGGAUGUAACUGAAUGAAAUGGUGAAGGACGGGUCCAG
UAGGCUGCUUCGGCAGCCUACUUGUUGAGUAGAGUGUGAGCUCCGUAACUAGUU
ACAUCCCCACAUACUCUGAUGAUCCNNNNGGAUCAUUCAUGGCAA
**SEQ-ID No: 60**
   F30::Broccoli 
UUGCCAUGUGUAUGUGGGAGACGGUCGGGUCCAGAUAUUCGUAUCUGUCGAGUA
GAGUGUGGGCUCCCACAUACUCUGAUGAUCCNNNNGGAUCAUUCAUGGCAA
**SEQ-ID No: 61**
   F30::Corn 
UUGCCAUGUGUAUGUGGGCGAGGAAGGAGGUCUGAGGAGGUCACUGCCCACAUA
CUCUGAUGAUCCNNNNGGAUCAUUCAUGGCAA
**SEQ-ID No: 62**
   F30::Orange broccoli 
UUGCCAUGUGUAUGUGGGAGACGGUCGGGUCCAGGUGCACAAAUGUGGCCUGUU
GAGUAGCGUGUGGGCUCCCACAUACUCUGAUGAUCCNNNNGGAUCAUUCAUGGCA
A
**SEQ-ID No: 63**
   F30::Red broccoli 
UUGCCAUGUGUAUGUGGGAGACGGUCGGGUCCAGUCCCAACGAUGUUGGCUGUU
GAGUAGUGUGUGGGCUCCCACAUACUCUGAUGAUCCNNNNGGAUCAUUCAUGGCA
A
**SEQ-ID No: 64**
   F30::Baby spinach 
UUGCCAUGUGUAUGUGGGAAGGACGGGUCCGUUGAGUAGAGUGUGAGCCCACAU
ACUCUGAUGAUCCNNNNGGAUCAUUCAUGGCAA
**SEQ-ID No: 77**
   F30-Broccoli-Broccoli 
UUGCCAUGUGUAUGUGGGAGACGGUCGGGUCCAGAUAUUCGUAUCUGUCGAGUA
GAGUGUGGGCUCCCACAUACUCUGAUGAUCCAGACGGUCGGGUCCAGAUAUUCG
UAUCUGUCGAGUAGAGUGUGGGCUGGAUCAUUCAUGGCAA
**SEQ-ID No: 78**
   F30-Broccoli-Mango

UUGCCAUGUGUAUGUGGGAGACGGUCGGGUCCAGAUAUUCGUAUCUGUCGAGUA
GAGUGUGGGCUCCCACAUACUCUGAUGAUCCGAAGGGACGGUGCGGAGAGGAGA
GGAUCAUUCAUGGCAA

The vector used in the invention can be introduced into the cells by any method known in the art, e.g. by transformation, transfection or viral transduction. The skilled person is aware how these methods can be further optimized to ensure that the vector is present in each cell in sufficient quantity. It is envisaged by the present invention that the vector can be integrated into the chromosome once it has been introduced into the cells. This integration may not necessarily include the complete vector sequence, but the sections of the vector required for expression of the target RNA in the given genetic context.

The method according to the invention aims at optimizing RNA production by identifying cells or culture conditions that increase RNA production. Therefore, in one embodiment, the cells used in the methods of the invention carry chromosomal genetic mutations that may influence RNA expression. The cells can be mutagenized using any technique known in the art, e.g., random mutagenesis via UV radiation or site-directed mutagenesis, for example via CRISPR-Cas.

In another embodiment, the cells harbor different vectors that are all capable of expressing the same RNA of interest, but differ in the other elements contained in the vector, so that the RNA yield from the vectors is different.

Once the vector has been introduced into the cells, the cells are cultured under conditions that allow expression of the heterologous RNA. In case that expression of the heterologous RNA of interest is conditional, the cells may first be cultured without inducing expression and expression be induced after some time.

In one embodiment, culture conditions between the cells are varied, for example with respect to temperature, dissolved oxygen level, stirring speed, pressure or culture medium. This embodiment allows to identify optimal culture conditions for the expression of the heterologous RNA in question. Therefore, the method of the invention can also be used to optimize culture conditions for the production of a particular RNA of interest.

After the cells have been cultured for a time sufficient to express the heterologous RNA from the vector, the fluorophore that is capable of binding the aptamer with which the heterologous RNA is tagged is added to the culture medium and allowed to enter the cells where it can bind to the aptamer. It is known in the art how to determine a suitable concentration of the fluorophore in the culture medium.

In those cells that have a high concentration of the heterologous RNA of interest, the tag is present in higher quantities and thus higher amounts of the fluorophore are bound which will then emit fluorescence. In contrast, those cells showing only a low concentration of the RNA of interest including the tag will harbor less activated fluorophore and therefore emit less fluorescence. Importantly, fluorophore that is present in the cell, but not bound to the aptamer, will exhibit only very weak or no fluorescence.

The degree of fluorescence emitted by each cell can be determined using any technique known in the art. In one embodiment, the cells are assessed by spectrometry. In a preferred embodiment, the cells are sorted according to their fluorescence level by flow cytometry. This allows to identify and, at the same time, isolate those cells showing a high level of fluorescence. Isolated cells may be subsequently analyzed for the chromosomal genetic alterations that they carry or genetic alterations in the vector. Likewise, it is possible to determine those culture conditions that yield the highest number of fluorescent cells.

In another aspect, the invention relates to a method for producing a RNA of interest, comprising the steps of
a) introducing into a plurality of cells a first vector capable of expressing a heterologous RNA of interest, wherein said RNA is tagged with a RNA tag comprising an aptamer capable of stabilizing a fluorophore and a scaffold capable of stabilizing the aptamer;
b) culturing the cells in a culture medium under conditions that allow expression of the heterologous RNA of interest;
c) adding said fluorophore to the culture medium;
d) identifying and isolating those cells that show the highest intensity of fluorescence and therefore a high expression of the RNA of interest;
e) removing the first vector of step a) from the cells isolated in step d);
f) introducing a second vector capable of expressing the heterologous RNA of interest without the RNA tag into the cells obtained in step e);
g) producing the RNA of interest by culturing the cells obtained in step f).

Producing a RNA of interest according to the method of the invention comprises first identifying cells that show a high expression of the RNA of interest with the help of the RNA tag and then using these cells for the production of the RNA of interest.

Because it is desirable that the final product does not include any unnecessary sequences, the vector capable of expressing the tagged RNA of interest is removed prior to RNA production once suitable cells have been identified. Removal of a vector can be achieved by preparation of electrocompetent cells as previously described (Tauch, A., Kirchner, O., Löffler, B., Götker, S., Pühler, A., and Kalinowski, J. Efficient Electrotransformation of Corynebacterium diphtheriae with a Mini-Replicon Derived from the Corynebacterium glutamicum Plasmid pGA1. Curr. Microbiol. 2002;45, 362-367) and electroporation of these cell without addition of a DNA template. This increases the likelihood of spontaneous vector loss. Cells are subsequently transformed with a second vector that is identical to the first vector except that the second vector does not contain the RNA tag. These cells are then used for producing the RNA of interest.

Extraction and, optionally, purification of the produced RNA can be performed according to methods known in the art. Likewise, the amount of produced RNA can be quantified after extraction using well-known techniques.

The method of the invention allows to maximize the production of the RNA of interest by specifically selecting cells that show a high expression of the RNA of interest. Because the vectors used in the invention can be easily engineered to carry any RNA of interest, the invention provides a fast, efficient and universally applicable way to save costs and time when producing a certain RNA molecule.

In a third aspect, the invention relates to a microbial cell harboring a vector capable of expressing a heterologous RNA of interest, wherein said RNA is tagged with a RNA tag comprising
- an aptamer capable of stabilizing a fluorophore and
- an RNA scaffold capable of stabilizing the aptamer.

The cells according to the invention harbor a vector capable of expressing a heterologous RNA of interest. "Harboring" is herein defined as meaning that the cells contain or comprise a vector either as an extrachromosomal plasmid or integrated into one or several of their chromosomes.

Because the heterologous RNA that is expressed by the cells of the invention is tagged, it can be detected and quantified once the corresponding fluorophore has been added to the cells. Therefore, cells according to the invention can be easily and conveniently classified and separated (e.g. by flow cytometry) based on the amount of heterologous RNA they produce.

### Examples

Hereinafter, the present invention is described in more detail with reference to Figures and the Examples, which however are not intended to limit the present invention.

### Example 1

### a) Construction of the vectors pJC1-PF1-U1A-F30::broccoli/UUCG-TF1 and pJC1-PF1-U1A-TF1

The construction of the plasmid **vector** was achieved by means of chemical synthesis of synthetic DNA-fragments (SEQ-ID No: 72 for pJC1-PF1-U1A-TF1 and SEQ-ID No: 71 for pJC1-PF1-U1A-F30::broccoli/UUCG-TF1) and their ligation into pJC1 (Cremer, J., Treptow, C., Eggeling, L., and Sahm, H. Regulation of Enzymes of Lysine Biosynthesis in Corynebacterium glutamicum. Microbiol. 1988; 134, 3221-3229). SEQ-ID No: 71 contained the promoter P_{F1} (SEQ-ID No: 67), the non-coding, recombinant U1A*-RNA (SEQ-ID No: 68) that was described earlier (Hashiro, S., Mitsuhashi, M., and Yasueda, H. Overexpression system for recombinant RNA in Corynebacterium glutamicum using a strong promoter derived from corynephage BFK20. J. Biosci. Bioeng. 2019;128, 255-263), the F30 scaffold (SEQ-ID No: 69) with a broccoli aptamer (SEQ-ID No: 70) in the first integration point and a "UUCG spacer" in the second integration point and a terminator sequence T_{F1} (SEQ-ID No: 70). SEQ-ID No: 72 contained the promoter P_{F1} (SEQ-ID No: 67), a the non-coding, recombinant U1A*-RNA (SEQ-ID No: 68) and a terminator sequence T_{F1} (SEQ-ID No: 70), but neither scaffold nor aptamer sequence.

After cleavage of the synthesized DNA fragments with the restriction enzymes *Xbal* and *Sa*/I and subsequent purification of the reaction mixtures, the DNA fragments that had been cut out were used in individual ligation reactions with vector pJC1 that had also been linearized with *Xba*I and *Sa*/I and dephosphorylated. The ligation mixtures were used directly to transform *E. coli* XL1-blue, and the selection of transformants was carried out on LB plates containing 50 µg/ml kanamycin. 16 colonies which grew on these plates and were therefore resistant to kanamycin were used for colony PCR. The colony PCR was performed with primers pJC1_check_f (SEQ-ID No: 73) and pJC1_check_rev (SEQ-ID No: 74), to analyze whether the synthesized fragments were inserted into vector pJC1. The analysis of colony PCR products in an agarose gel showed the expected PCR product with a size of 521 bp (pJC1-PF1-U1A-TF1) and 626 bp (pJC1-PF1-U1A-F30::broccoli/UUCG-TF1) in the samples that were analyzed, whereupon four colonies were cultured for plasmid preparations in a larger scale. After 16 h of cultivation these cultures were collected by centrifugation and plasmid DNA was prepared. Two of the plasmid preparations were sequenced with the primers used in the colony PCR. Sequence analysis of the inserts showed 100% identity with the expected sequence. The resulting plasmid were named pJC1-PF1-U1A-TF1 (SEQ-ID No: 76) and pJC1-PF1-U1A-F30::broccoli/UUCG-TF1 (SEQ-ID No: 75), respectively.
**SEQ ID No: 67**
   PF1: 
CTCGAGCGGGACGGTCGAACCAGCTTCAAGCGACCGGATGAGTATGTTACAGTAGA
TAGCG
**SEQ-ID No. 68**
   target RNA: 
AGCGGGAGACCGCTCGACCTTAGTTCTCCTGTTGCGGGGGAGTTCATGGGATCCAG
GTACCCAGGGCGAGGCTTATCCATTGCACTCCGGATGTGCTGACCCCTG
**SEQ-ID No: 69**
   F30 with broccoli in insertion site 1 and "TTCG" in insertion site 2: 
TTGCCATGTGTATGTGGGAGACGGTCGGGTCCAGATATTCGTATCTGTCGAGTAGAG
TGTGGGCTCCCACATACTCTGATGATCCTTCGGGATCATTCATGGCAA
**SEQ-ID No: 70**
   TF1: 
GCTAGCATAGCATAAAATAACGCCCCACCTTCTTAACGGAGGTGGGGCGTTATTTT
TACGGGGATCGCTTCTTCCGCTTCCTCGCTCACTGACTCGCTGCGCTCGGTCGTTCG
GCTGCGG
**SEQ-ID No: 71**
   SEQ-ID No: 67-70 combined, with Xbal recognition site on 5'-end, SaII recognition site on 3'-end: 
CTGTCTCTAGACTCGAGCGGACGGTCGAACCAGCTTCAAGCGACCGGATGAGTAT
GTTACAGTAGATAGCGAGCGGAGACCGCTCGACCTTAGTTCTCCTGTTGCGGGG
AGTTCATGGGATCCAGGTACCCAGGGCGAGGCTTATCCATTGCACTCCGGATGTGC
TGACCCCTGTTGCCATGTGTATGTGGAGACGGTCGGGTCCAGATATTCGTATCTGT
CGAGTAGAGTGTGGGCTCCCACATACTCTGATGATCCTTCGGGATCATTCATGGCAA
GCTAGCATAGCATAAAATAACGCCCCACCTTCTTAACGGAGGTGGGGCGTTATTTT
TACGGGGATCGCTTCTTCCGCTTCCTCGCTCACTGACTCGCTGCGCTCGGTCGTTCG
GCTGCGGGTCGACCTGTC
**SEQ-ID No: 72**
   SEQ-ID No: 67, 68 and 70, with Xbal recognition site on 5'-end, SaII recognition site on 3'-end: 
CTGTCTCTAGACTCGAGCGGACGGTCGAACCAGCTTCAAGCGACCGGATGAGTAT
GTTACAGTAGATAGCGAGCGGAGACCGCTCGACCTTAGTTCTCCTGTTGCGGGG
AGTTCATGGGATCCAGGTACCCAGGGCGAGGCTTATCCATTGCACTCCGGATGTGC
TGACCCCTGGCTAGCATAGCATAAAATAACGCCCCACCTTCTTAACGGGAGGTGGG
 
GCGTTATTTTTACGGGGATCGCTTCTTCCGCTTCCTCGCTCACTGACTCGCTGCGCT
CGGTCGTTCGGCTGCGGGTCGACCTGTC
**SEQ-ID No: 73**
   pJC1_check_f:
   TGAAGACCGTCAACCAAAGG
**SEQ-ID No: 74**
   pJC1_check_rev:
   TGCCGGGAAGCTAGAGTAAG
**SEQ-ID No: 75**
   pJC1-PF1-U1A-F30::broccoli/UUCG-TF1 
CCGAGAAGTTTTTACAAAGGCAAAAACTTTTTCGGGATCGACAGAAATAAAACGAT
CGACGGTACGCAACAAAAAAGCGTCAGGATCGCCGTAGAGCGATTGAAGACCGTCA
ACCAAAGGGGAAGCCTCCAATCGACGCGACGCGCGCTCTACGGCGATCCTGACGCA
GATTTTTAGCTATCTGTCGCAGCGCCCTCAGGGACAAGCCACCCGCACAACGTCGC
GAGGGCGATCAGCGACGCCGCAGGGGGATCCTCTAGACTCGAGCGGACGGTCGA
ACCAGCTTCAAGCGACCGGATGAGTATGTTACAGTAGATAGCGAGCGGGAGACCGC
TCGACCTTAGTTCTCCTGTTGCGGGGGAGTTCATGGGATCCAGGTACCCAGGGCGA
GGCTTATCCATTGCACTCCGGATGTGCTGACCCCTGTTGCCATGTGTATGTGGAGA
CGGTCGGGTCCAGATATTCGTATCTGTCGAGTAGAGTGTGGGCTCCCACATACTCTG
ATGATCCTTCGGGATCATTCATGGCAAGCTAGCATAGCATAAAATAACGCCCCACCTT
CTTAACGGGAGGTGGGGCGTTATTTTACGGGGATCGCTTCTTCCGCTTCCTCGCTC
ACTGACTCGCTGCGCTCGGTCGTTCGGCTGCGGGTCGACCTGCAGCAATGGCAACA
ACGTTGCGCAAACTATTAACTGGCGAACTACTTACTCTAGCTTCCCGGCAACAATTAA
TAGACTGGATGGAGGCGGATAAAGTTGCAGGACCACTTCTGCGCTCGGCCCTTCCG
GCTGGCTGGTTTATTGCTGATAAATCTGGAGCCGGTGAGCGTGGGTCTCGCGGTAT
CATTGCAGCACTGGGGCCAGATGGTAAGCCCTCCCGTATCGTAGTTATCTACACGAC
GGGGAGTCAGGCAACTATGGATGAACGAAATAGACAGATCGCTGAGATAGGTGCCT
CACTGATTAAGCATTGGTAACTGTCAGACCAAGTTTACTCATATATACTTTAGATTGAT
TTAAAACTTCATTTTTAATTTAAAAGGATCTAGGTGAAGATCCTTTTTGATAATCTCATG
ACCAAAATCCCTTAACGTGAGTTTTCGTTCCACTGAGCGTCAGACCCCTTAATAAGAT
GATCTTCTTGAGATCGTTTTGGTCTGCGCGTAATCTCTTGCTCTGAAAACGAAAAAAC
CGCCTTGCAGGGCGGTTTTTCGAAGGTTCTCTGAGCTACCAACTCTTTGAACCGAGG
TAACTGGCTTGGAGGAGCGCAGTCACCAAAACTTGTCCTTTCAGTTTAGCCTTAACC
 
GGCGCATGACTTCAAGACTAACTCCTCTAAATCAATTACCAGTGGCTGCTGCCAGTG
GTGCTTTTGCATGTCTTTCCGGGTTGGACTCAAGACGATAGTTACCGGATAAGGCGC
AGCGGTCGGACTGAACGGGGGGTTCGTGCATACAGTCCAGCTTGGAGCGAACTGCC
TACCCGGAACTGAGTGTCAGGCGTGGAATGAGACAAACGCGGCCATAACAGCGGAA
TGACACCGGTAAACCGAAAGGCAGGAACAGGAGAGCGCACGAGGGAGCCGCCAGG
GGGAAACGCCTGGTATCTTTATAGTCCTGTCGGGTTTCGCCACCACTGATTTGAGCG
TCAGATTTCGTGATGCTTGTCAGGGGGGCGGAGCCTATGGAAAAACGGCTTTGCCG
CGGCCCTCTCACTTCCCTGTTAAGTATCTTCCTGGCATCTTCCAGGAAATCTCCGCC
CCGTTCGTAAGCCATTTCCGCTCGCCGCAGTCGAACGACCGAGCGTAGCGAGTCAG
TGAGCGAGGAAGCGGAATATATCCTGTATCACATATTCTGCTGACGCACCGGTGCAG
CCTTTTTTCTCCTGCCACATGAAGCACTTCACTGACACCCTCATCAGTGCCAACATAG
TAAGCCAGTATACACTCCGCTAGCGCTGAGGTCTGCCTCGTGAAGAAGGTGTTGCTG
ACTCATACCAGGCCTGAATCGCCCCATCATCCAGCCAGAAAGTGAGGGAGCCACGG
TTGATGAGAGCTTTGTTGTAGGTGGACCAGTTGGTGATTTTGAACTTTTGCTTTGCCA
CGGAACGGTCTGCGTTGTCGGGAAGATGCGTGATCTGATCCTTCAACTCAGCAAAAG
TTCGATTTATTCAACAAAGCCACGTTGTGTCTCAAAATCTCTGATGTTACATTGCACAA
GATAAAAATATATCATCATGAACAATAAAACTGTCTGCTTACATAAACAGTAATACAAG
GGGTGTTATGAGCCATATTCAACGGGAAACGTCTTGCTCGAGGCCGCGATTAAATTC
CAACATGGATGCTGATTTATATGGGTATAAATGGGCTCGCGATAATGTCGGGCAATC
AGGTGCGACAATCTATCGATTGTATGGGAAGCCCGATGCGCCAGAGTTGTTTCTGAA
ACATGGCAAAGGTAGCGTTGCCAATGATGTTACAGATGAGATGGTCAGACTAAACTG
GCTGACGGAATTTATGCCTCTTCCGACCATCAAGCATTTTATCC
GTACTCCTGATGATGCATGGTTACTCACCACTGCGATCCCCGGAAAACA
GCATTCCAGGTATTAGAAGAATATCCTGATTCAGGTGAAAATATTGTTGA
TGCGCTGGCAGTGTTCCTGCGCCGGTTGCATTCGATTCCTGTTTGTAATT
GTCCTTTTAACAGCGATCGCGTATTTCGTCTCGCTCAGGCGCAATCACGA
ATGAATAACGGTTTGGTTGATGCGAGTGATTTTGATGACGAGCGTAATGG
CTGGCCTGTTGAACAAGTCTGGAAAGAAATGCATAAGCTTTTGCCATTCT
CACCGGATTCAGTCGTCACTCATGGTGATTTCTCACTTGATAACCTTATT
TTTGACGAGGGGAAATTAATAGGTTGTATTGATGTTGGACGAGTCGGAAT
CGCAGACCGATACCAGGATCTTGCCATCCTATGGAACTGCCTCGGTGAGT
TTTCTCCTTCATTACAGAAACGGCTTTTTCAAAAATATGGTATTGATAAT
CCTGATATGAATAAATTGCAGTTTCATTTGATGCTCGATGAGTTTTTCTA
ATCAGAATTGGTTAATTGGTTGTAACACTGGCAGAGCATTACGCTGACTT
GACGGGACGGCGGCTTTGTTGAATAAATCGAACTTTTGCTGAGTTGAAGG
ATCAGATCACGCATCTTCCCGACAACGCAGACCGTTCCGTGGCAAAGCAA
 
AAGTTCAAAATCACCAACTGGTCCACCTACAACAAAGCTCTCATCAACCG
TGGCTCCCTCACTTTCTGGCTGGATGATGGGGCGATTCAGGCCTGGTATG
AGTCAGCAACACCTTCTTCACGAGGCAGACCTCAGCGCTCAAAGATGCAG
GGGTAAAAGCTAACCGCATCTTTACCGACAAGGCATCCGGCAGTTCAACA
GATCGGGAAGGGCTGGATTTGCTGAGGATGAAGGTGGAGGAAGGTGATGT
CATTCTGGTGAAGAAGCTCGACCGTCTTGGCCGCGACACCGCCGACATGA
TCCAACTGATAAAAGAGTTTGATGCTCAGGGTGTAGCGGTTCGGTTTATT
GACGACGGGATCAGTACCGACGGTGATATGGGGCAAATGGTGGTCACCAT
CCTGTCGGCTGTGGCACAGGCTGAACGCCGGAGGATCAAGTCGGTCAAGC
CAAGCGCAACCAGCGGCACCGCCGCGAGCAACGTCGCAAGGGCGATCAGG
GGACGATTTTTGCGAAGAATTTCCACGGTAAGAATCCAATCTCTCGAATT
TAGGGTGAAAGAAGCTTGGCATAGGGGTGTGCACGAACTCGGTGGAGGAA
ATTTCCGCGGGGCAAGGCTTCGCGAAGCGGAGTCGCGGCAGTGGCTTTGA
AGATCTTTGGGAGCAGTCCTTGTGCGCTTACGAGGTGAGCCGGTGGGGAA
CCGTTATCTGCCTATGGTGTGAGCCCCCCTAGAGAGCTTCAAGAGCAATC
AGCCCGACCTAGAAAGGAGGCCAAGAGAGAGACCCCTACGGGGGGAACCG
TTTTCTGCCTACGAGATGGCACATTTACTGGGAAGCTTTACGGCGTCCTC
GTGGAAGTTCAATGCCCGCAGACTTAAGTGCTCTATTCACGGTCTGACGT
GACACGCTAAATTCAGACATAGCTTCATTGATTGTCGGCCACGAGCCAGT
CTCTCCCTCAACAGTCATAAACCAACCTGCAATGGTCAAGCGATTTCCTT
TAGCTTTCCTAGCTTGTCGTTGACTGGACTTAGCTAGTTTTTCTCGCTGT
GCTCGGGCGTACTCACTGTTTGGGTCTTTCCAGCGTTCTGCGGCCTTTTT
ACCGCCACGTCTTCCCATAGTGGCCAGAGCTTTTCGCCCTCGGCTGCTCT
GCGTCTCTGTCTGACGAGCAGGGACGACTGGCTGGCCTTTAGCGACGTAG
CCGCGCACACGTCGCGCCATCGTCTGGCGGTCACGCATCGGCGGCAGATC
AGGCTCACGGCCGTCTGCTCCGACCGCCTGAGCGACGGTGTAGGCACGCT
CGTAGGCGTCGATGATCTTGGTGTCTTTTAGGCGCTCACCAGCCGCTTTT
AACTGGTATCCCACAGTCAAAGCGTGGCGAAAAGCCGTCTCATCACGGGC
GGCACGCCCTGGAGCAGTCCAGAGGACACGGACGCCGTCGATCAGCTCTC
CAGACGCTTCAGCGGCGCTCGGCAGGCTTGCTTCAAGCGTGGCAAGTGCT
TTTGCTTCCGCAGTGGCTTTTCTTGCCGCTTCGATACGTGCCCGTCCGCT
AGAAAACTCCTGCTCATAGCGTTTTTTAGGTTTTTCTGTGCCTGAGATCA
TGCGAGCAACCTCCATAAGATCAGCTAGGCGATCCACGCGATTGTGCTGG
GCATGCCAGCGGTACGCGGTGGGATCGTCGGAGACGTGCAGTGGCCACCG
GCTCAGCCTATGTGAAAAAGCCTGGTCAGCGCCGAAAACGCGGGTCATTT
CCTCGGTCGTTGCAGCCAGCAGGCGCATATTCGGGCTGCTCATGCCTGCT
 
GCGGCATACACCGGATCAATGAGCCAGATGAGCTGGCATTTCCCGCTCAG
TGGATTCACGCCGATCCAAGCTGGCGCTTTTCCAGGCGTGCCCAGCGCT
CCAAAATCGCGTAGACCTCGGGGTTTACGTGCTCGATTTTCCCGCCGGCC
TGGTGGCTCGGCACATCAATGTCCAGGACAAGCACGGCTGCGTGCTGCGC
GTGCGTCAGAGCAACATACTGGCACCGGGCAAGCGATTTTGAACCAACTC
GGTATAACTTCGGCTGTGTTTCTCCCGTGTCCGGGTCTTTGATCCAAGCG
CTGGCGAAGTCGCGGGTCTTGCTGCCCTGGAAATTTTCTCTGCCCAGGTG
AGCGAGGAATTCGCGGCGGTCTTCGCTCGTCCAGCCACGTGATCGCAGCG
CGAGCTCGGGATGGGTGTCGAACAGATCAGCGGAAAATTTCCAGGCCGGT
GTGTCAATGTCTCGTGAATCCGCTAGAGTCATTTTTGAGCGCTTTCTCCC
AGGTTTGGACTGGGGGTTAGCCGACGCCCTGTGAGTTACCGCTCACGGGG
CGTTCAACATTTTTCAGGTATTCGTGCAGCTTATCGCTTCTTGCCGCCTG
TGCGCTTTTTCGACGCGCGACGCTGCTGCCGATTCGGTGCAGGTGGTGGC
GGCGCTGACACGTCCTGGGCGGCCACGGCCACACGAAACGCGGCATTTAC
GATGTTTGTCATGCCTGCGGGCACCGCGCCACGATCGCGGATAATTCTCG
CTGCCGCTTCCAGCTCTGTGACGACCATGGCCAAAATTTCGCTCGGGGGA
CGCACTTCCAGCGCCATTTGCGACCTAGCCGCCTCCAGCTCCTCGGCGTG
GCGTTTGTTGGCGCGCTCGCGGCTGGCTGCGGCACGACACGCATCTGAGC
AATATTTTGCGCGCCGTCCTCGCGGGTCAGGCCGGGGAGGAATCAGGCCA
CCGCAGTAGGCGCAACTGATTCGATCCTCCACTACTGTGCGTCCTCCTGG
CGCTGCCGAGCACGCAGCTCGTCAGCCAGCTCCTCAAGATCCGCCACGAG
AGTTTCTAGGTCGCTCGCGGCACTGGCCCAGTCTCGTGATGCTGGCGCGT
CCGTCGTATCGAGAGCTCGGAAAAATCCGATCACCGTTTTTAAATCGACG
GCAGCATCGAGCGCGTCGGACTCCAGCGCGACATCAGAGAGATCCATAGC
TGATGATTCGGGCCAATTTTGGTACTTCGTCGTGAAGGTCATGACACCAT
TATAACGAACGTTCGTTAAAGTTTTTGGCGGAAAATCACGCGGCACGAAA
ATTTTCACGAAGCGGGACTTTGCGCAGCTCAGGGGTGCTAAAAATTTTGT
ATCGCACTTGATTTTTCCGAAAGACAGATTATCTGCAAACGGTGTGTCGT
ATTTCTGGCTTGGTTTTTAAAAAATCTGGAATCGAAAATTTGCGGGGCGA
**SEQ-ID No: 76**
   pJC1-PF1-U1A-TF1:

CCGAGAAGTTTTTTACAAAAGGCAAAAACTTTTTCGGGATCGACAGAAAT
AAAACGATCGACGGTACGCAACAAAAAAGCGTCAGGATCGCCGTAGAGCG
ATTGAAGACCGTCAACCAAAGGGGAAGCCTCCAATCGACGCGACGCGCGC
TCTACGGCGATCCTGACGCAGATTTTTAGCTATCTGTCGCAGCGCCCTCA

GGGACAAGCCACCCGCACAACGTCGCGAGGGCGATCAGCGACGCCGCAGG
GGGATCCTCTAGACTCGAGCGGGACGGTCGAACCAGCTTCAAGCGACCGG
ATGAGTATGTTACAGTAGATAGCGAGCGGAGACCGCTCGACCTTAGTTC
TCCTGTTGCGGGGGAGTTCATGGATCCAGGTACCCAGGGCGAGGCTTAT
CCATTGCACTCCGGATGTGCTGACCCCTGGCTAGCATAGCATAAAATAAC
GCCCCACCTTCTTAACGGGAGGTGGGGCGTTATTTTACGGGGATCGCTT
CTTCCGCTTCCTCGCTCACTGACTCGCTGCGCTCGGTCGTTCGGCTGCGG
GTCGACCTGCAGCAATGGCAACAACGTTGCGCAAACTATTAACTGGCGAA
CTACTTACTCTAGCTTCCCGGCAACAATTAATAGACTGGATGGAGGCGGA
TAAAGTTGCAGGACCACTTCTGCGCTCGGCCCTTCCGGCTGGCTGGTTTA
TTGCTGATAAATCTGGAGCCGGTGAGCGTGGGTCTCGCGGTATCATTGCA
GCACTGGGGCCAGATGGTAAGCCCTCCCGTATCGTAGTTATCTACACGAC
GGGGAGTCAGGCAACTATGGATGAACGAAATAGACAGATCGCTGAGATAG
GTGCCTCACTGATTAAGCATTGGTAACTGTCAGACCAAGTTTACTCATAT
ATACTTTAGATTGATTTAAAACTTCATTTTTAATTTAAAAGGATCTAGGT
GAAGATCCTTTTTGATAATCTCATGACCAAAATCCCTTAACGTGAGTTTT
CGTTCCACTGAGCGTCAGACCCCTTAATAAGATGATCTTCTTGAGATCGT
TTTGGTCTGCGCGTAATCTCTTGCTCTGAAAACGAAAAAACCGCCTTGCA
GGGCGGTTTTTCGAAGGTTCTCTGAGCTACCAACTCTTTGAACCGAGGTA
ACTGGCTTGGAGGAGCGCAGTCACCAAAACTTGTCCTTTCAGTTTAGCCT
TAACCGGCGCATGACTTCAAGACTAACTCCTCTAAATCAATTACCAGTGG
CTGCTGCCAGTGGTGCTTTTGCATGTCTTTCCGGGTTGGACTCAAGACGA
TAGTTACCGGATAAGGCGCAGCGGTCGGACTGAACGGGGGGTTCGTGCAT
ACAGTCCAGCTTGGAGCGAACTGCCTACCCGGAACTGAGTGTCAGGCGTG
GAATGAGACAAACGCGGCCATAACAGCGGAATGACACCGGTAAACCGAAA
GGCAGGAACAGGAGAGCGCACGAGGGAGCCGCCAGGGGGAAACGCCTGGT
ATCTTTATAGTCCTGTCGGGTTTCGCCACCACTGATTTGAGCGTCAGATT
TCGTGATGCTTGTCAGGGGGGCGGAGCCTATGGAAAAACGGCTTTGCCGC
GGCCCTCTCACTTCCCTGTTAAGTATCTTCCTGGCATCTTCCAGGAAATC
TCCGCCCCGTTCGTAAGCCATTTCCGCTCGCCGCAGTCGAACGACCGAGC
GTAGCGAGTCAGTGAGCGAGGAAGCGGAATATATCCTGTATCACATATTC
TGCTGACGCACCGGTGCAGCCTTTTTTCTCCTGCCACATGAAGCACTTCA
CTGACACCCTCATCAGTGCCAACATAGTAAGCCAGTATACACTCCGCTAG
CGCTGAGGTCTGCCTCGTGAAGAAGGTGTTGCTGACTCATACCAGGCCTG
AATCGCCCCATCATCCAGCCAGAAAGTGAGGGAGCCACGGTTGATGAGAG
CTTTGTTGTAGGTGGACCAGTTGGTGATTTTGAACTTTTGCTTTGCCACG

GAACGGTCTGCGTTGTCGGGAAGATGCGTGATCTGATCCTTCAACTCAGC
AAAAGTTCGATTTATTCAACAAAGCCACGTTGTGTCTCAAAATCTCTGAT
GTTACATTGCACAAGATAAAAATATATCATCATGAACAATAAAACTGTCT
GCTTACATAAACAGTAATACAAGGGGTGTTATGAGCCATATTCAACGGGA
AACGTCTTGCTCGAGGCCGCGATTAAATTCCAACATGGATGCTGATTTAT
ATGGGTATAAATGGGCTCGCGATAATGTCGGGCAATCAGGTGCGACAATC
TATCGATTGTATGGGAAGCCCGATGCGCCAGAGTTGTTTCTGAAACATGG
CAAAGGTAGCGTTGCCAATGATGTTACAGATGAGATGGTCAGACTAAACT
GGCTGACGGAATTTATGCCTCTTCCGACCATCAAGCATTTTATCCGTACT
CCTGATGATGCATGGTTACTCACCACTGCGATCCCCGGGAAAACAGCATT
CCAGGTATTAGAAGAATATCCTGATTCAGGTGAAAATATTGTTGATGCGC
TGGCAGTGTTCCTGCGCCGGTTGCATTCGATTCCTGTTTGTAATTGTCCT
TTTAACAGCGATCGCGTATTTCGTCTCGCTCAGGCGCAATCACGAATGAA
TAACGGTTTGGTTGATGCGAGTGATTTTGATGACGAGCGTAATGGCTGGC
CTGTTGAACAAGTCTGGAAAGAAATGCATAAGCTTTTGCCATTCTCACCG
GATTCAGTCGTCACTCATGGTGATTTCTCACTTGATAACCTTATTTTTGA
CGAGGGGAAATTAATAGGTTGTATTGATGTTGGACGAGTCGGAATCGCAG
ACCGATACCAGGATCTTGCCATCCTATGGAACTGCCTCGGTGAGTTTTCT
CCTTCATTACAGAAACGGCTTTTTCAAAAATATGGTATTGATAATCCTGA
TATGAATAAATTGCAGTTTCATTTGATGCTCGATGAGTTTTTCTAATCAG
AATTGGTTAATTGGTTGTAACACTGGCAGAGCATTACGCTGACTTGACGG
GACGGCGGCTTTGTTGAATAAATCGAACTTTTGCTGAGTTGAAGGATCAG
ATCACGCATCTTCCCGACAACGCAGACCGTTCCGTGGCAAAGCAAAAGTT
CAAAATCACCAACTGGTCCACCTACAACAAAGCTCTCATCAACCGTGGCT
CCCTCACTTTCTGGCTGGATGATGGGGCGATTCAGGCCTGGTATGAGTCA
GCAACACCTTCTTCACGAGGCAGACCTCAGCGCTCAAAGATGCAGGGGTA
AAAGCTAACCGCATCTTTACCGACAAGGCATCCGGCAGTTCAACAGATCG
GGAAGGGCTGGATTTGCTGAGGATGAAGGTGGAGGAAGGTGATGTCATTC
TGGTGAAGAAGCTCGACCGTCTTGGCCGCGACACCGCCGACATGATCCAA
CTGATAAAAGAGTTTGATGCTCAGGGTGTAGCGGTTCGGTTTATTGACGA
CGGGATCAGTACCGACGGTGATATGGGGCAAATGGTGGTCACCATCCTGT
CGGCTGTGGCACAGGCTGAACGCCGGAGGATCAAGTCGGTCAAGCCAAGC
GCAACCAGCGGCACCGCCGCGAGCAACGTCGCAAGGGCGATCAGGGGACG
ATTTTTGCGAAGAATTTCCACGGTAAGAATCCAATCTCTCGAATTTAGGG
TGAAAGAAGCTTGGCATAGGGGTGTGCACGAACTCGGTGGAGGAAATTTC
CGCGGGGCAAGGCTTCGCGAAGCGGAGTCGCGGCAGTGGCTTTGAAGATC

TTTGGGAGCAGTCCTTGTGCGCTTACGAGGTGAGCCGGTGGGAACCGTT
ATCTGCCTATGGTGTGAGCCCCCCTAGAGAGCTTCAAGAGCAATCAGCCC
GACCTAGAAAGGAGGCCAAGAGAGACCCCTACGGGGGAACCGTTTTC
TGCCTACGAGATGGCACATTTACTGGGAAGCTTTACGGCGTCCTCGTGGA
AGTTCAATGCCCGCAGACTTAAGTGCTCTATTCACGGTCTGACGTGACAC
GCTAAATTCAGACATAGCTTCATTGATTGTCGGCCACGAGCCAGTCTCTC
CCTCAACAGTCATAAACCAACCTGCAATGGTCAAGCGATTTCCTTTAGCT
TTCCTAGCTTGTCGTTGACTGGACTTAGCTAGTTTTTCTCGCTGTGCTCG
GGCGTACTCACTGTTTGGGTCTTTCCAGCGTTCTGCGGCCTTTTTACCGC
CACGTCTTCCCATAGTGGCCAGAGCTTTTCGCCCTCGGCTGCTCTGCGTC
TCTGTCTGACGAGCAGGGACGACTGGCTGGCCTTTAGCGACGTAGCCGCG
CACACGTCGCGCCATCGTCTGGCGGTCACGCATCGGCGGCAGATCAGGCT
CACGGCCGTCTGCTCCGACCGCCTGAGCGACGGTGTAGGCACGCTCGTAG
GCGTCGATGATCTTGGTGTCTTTTAGGCGCTCACCAGCCGCTTTTAACTG
GTATCCCACAGTCAAAGCGTGGCGAAAAGCCGTCTCATCACGGGCGGCAC
GCCCTGGAGCAGTCCAGAGGACACGGACGCCGTCGATCAGCTCTCCAGAC
GCTTCAGCGGCGCTCGGCAGGCTTGCTTCAAGCGTGGCAAGTGCTTTTGC
TTCCGCAGTGGCTTTTCTTGCCGCTTCGATACGTGCCCGTCCGCTAGAAA
ACTCCTGCTCATAGCGTTTTTTAGGTTTTTCTGTGCCTGAGATCATGCGA
GCAACCTCCATAAGATCAGCTAGGCGATCCACGCGATTGTGCTGGGCATG
CCAGCGGTACGCGGTGGGATCGTCGGAGACGTGCAGTGGCCACCGGCTCA
GCCTATGTGAAAAAGCCTGGTCAGCGCCGAAAACGCGGGTCATTTCCTCG
GTCGTTGCAGCCAGCAGGCGCATATTCGGGCTGCTCATGCCTGCTGCGGC
ATACACCGGATCAATGAGCCAGATGAGCTGGCATTTCCCGCTCAGTGGAT
TCACGCCGATCCAAGCTGGCGCTTTTTCCAGGCGTGCCCAGCGCTCCAAA
ATCGCGTAGACCTCGGGGTTTACGTGCTCGATTTTCCCGCCGGCCTGGTG
GCTCGGCACATCAATGTCCAGGACAAGCACGGCTGCGTGCTGCGCGTGCG
TCAGAGCAACATACTGGCACCGGGCAAGCGATTTTGAACCAACTCGGTAT
AACTTCGGCTGTGTTTCTCCCGTGTCCGGGTCTTTGATCCAAGCGCTGGC
GAAGTCGCGGGTCTTGCTGCCCTGGAAATTTTCTCTGCCCAGGTGAGCGA
GGAATTCGCGGCGGTCTTCGCTCGTCCAGCCACGTGATCGCAGCGCGAGC
TCGGGATGGGTGTCGAACAGATCAGCGGAAAATTTCCAGGCCGGTGTGTC
AATGTCTCGTGAATCCGCTAGAGTCATTTTTGAGCGCTTTCTCCCAGGTT
TGGACTGGGGGTTAGCCGACGCCCTGTGAGTTACCGCTCACGGGGCGTTC
AACATTTTTCAGGTATTCGTGCAGCTTATCGCTTCTTGCCGCCTGTGCGC
TTTTTCGACGCGCGACGCTGCTGCCGATTCGGTGCAGGTGGTGGCGGCGC

TGACACGTCCTGGGCGGCCACGGCCACACGAAACGCGGCATTTACGATGT
TTGTCATGCCTGCGGGCACCGCGCCACGATCGCGGATAATTCTCGCTGCC
GCTTCCAGCTCTGTGACGACCATGGCCAAAATTTCGCTCGGGGGACGCAC
TTCCAGCGCCATTTGCGACCTAGCCGCCTCCAGCTCCTCGGCGTGGCGTT
TGTTGGCGCGCTCGCGGCTGGCTGCGGCACGACACGCATCTGAGCAATAT
TTTGCGCGCCGTCCTCGCGGGTCAGGCCGGGGAGGAATCAGGCCACCGCA
GTAGGCGCAACTGATTCGATCCTCCACTACTGTGCGTCCTCCTGGCGCTG
CCGAGCACGCAGCTCGTCAGCCAGCTCCTCAAGATCCGCCACGAGAGTTT
CTAGGTCGCTCGCGGCACTGGCCCAGTCTCGTGATGCTGGCGCGTCCGTC
GTATCGAGAGCTCGGAAAAATCCGATCACCGTTTTTAAATCGACGGCAGC
ATCGAGCGCGTCGGACTCCAGCGCGACATCAGAGAGATCCATAGCTGATG
ATTCGGGCCAATTTTGGTACTTCGTCGTGAAGGTCATGACACCATTATAA
CGAACGTTCGTTAAAGTTTTTGGCGGAAAATCACGCGGCACGAAAATTTT
CACGAAGCGGGACTTTGCGCAGCTCAGGGGTGCTAAAAATTTTGTATCGC
ACTTGATTTTTCCGAAAGACAGATTATCTGCAAACGGTGTGTCGTATTTC
TGGCTTGGTTTTTAAAAAATCTGGAATCGAAAATTTGCGGGGCGA

### b) Transformation of Corynebacterium glutamicum with pJC1-PF1-U1A-F30::broccoli/UUCG-TF1 and pJC1-PF1-U1A-TF1

Competent cells of the *C. glutamicum* strain ATCC 13032 *Δcg2273* were prepared and transformed with pJC1 PF1 U1A F30::broccoli/UUCG-TF1 and pJC1-PF1-U1A-TF1 according to Tauch *et al.,* 2002 (Tauch, A., Kirchner, O., Löffler, B., Götker, S., Pühler, A., and Kalinowski, J. Efficient Electrotransformation of Corynebacterium diphtheriae with a Mini-Replicon Derived from the Corynebacterium glutamicum Plasmid pGA1. Curr. Microbiol. 2002;45, 362-367). The selection of the transformants was carried out on CGIII (Menkel, E., Thierbach, G., Eggeling, L., and Sahm, H. Influence of increased aspartate availability on lysine formation by a recombinant strain of Corynebacterium glutamicum and utilization of fumarate. Appl. Environ. Microbiol. 1989;55, 684-688) agar (1 %) plates with 15 µg/ml of kanamycin. Clones thus obtained were named *C. glutamicum* ATCC 13032 Δcg2273 pJC1-PF1-U1A-F30::broccoli/UUCG-TF1 or *C. glutamicum* ATCC 13032 Δcg2273 pJC1-PF1-U1A-TF1, depending on which plasmid was used for transformation.

### c) Mutagenesis of C. glutamicum ATCC 13032 Δcg2273 pJC1-PF1-U1A-F30::broccoli/UUCG-TF1

The produced strain *C. glutamicum* ATCC 13032 *Δcg2273* pJC1-PF1-U1A-F30::broccoli/UUCG-TF1 was cultured overnight in CGIII medium at 30 °C, 120 rpm, 10 mL total volume with 15 µg/mL kanamycin added to the medium. Cells from this preculture were used to prepare a cell suspension with an OD₆₀₀ of 0,5 in 5 mL total volume of phosphate-buffered-saline (PBS). N-Methyl-N'-nitro-N-nitrosoguanidine (MNNG) was added to a final concentration of 25 µg/mL. After 20 min of incubation, a 1.5 mL sample was taken, centrifuged (2000 rpm, 2 min) and resuspended in 2 mL PBS. This washing step was repeated twice prior to final resuspension in 1.5 mL PBS and transfer into 15 mL fresh CGIII cultivation medium with 15 µg/mL kanamycin. The culture was subjected to a 16-hour cultivation at 30 °C, 120 rpm in a non-baffled shake flask.

### d) Identification and isolation of cells that show the highest intensity of fluorescence and therefore a high expression of the RNA of interest by the means of Fluorescence-activated cell sorting (FACS)

The regeneration culture from c) was diluted to an OD of 0.6 using PBS with a final concentration of 500 µM DFHBI. After 10 min of incubation, the cell suspension was analyzed in an Arialll High-speed cell sorter (BD Biosciences, Franklin Lakes, NJ, USA) equipped with a 70 µm nozzle and run with a sheath pressure of 70 psi. A 488 nm blue solid laser was used for excitation. Forward-scatter characteristics (FSC) were recorded as small-angle scatter and side-scatter characteristics (SSC) were recorded as orthogonal scatter of the 488 nm laser. A 502 nm long-pass and 530/30 nm band-pass filter combination were used for fluorescence detection. FACSDiva 8.0.1 (BD Biosciences, San Jose, USA) was used for FACS control and data analysis. Prior to data acquisition, debris and electronic noise were excluded from the analysis by electronic gating in the FSC-H against SSC-H plot. Another gating step was performed on the resulting population in the FSC-H against FSC-W plot to exclude doublets. Fluorescence acquisition was performed with the population resulting from this two-step gating (Fig. 1). 96 cells that exhibited an increased fluorescence in comparison to *C. glutamicum* ATCC 13032 *Δcg2273* pJC1-PF1-U1A-F30::broccoli/UUCG-TF1 without MNNG treatment were isolated from the culture broth and placed on CGIII agar plates (1%) with 15 µg/mL kanamycin.

### e) Cultivation of cells isolated in d) for phenotype validation

Of the isolated cells, 19 grew into colonies within the next 48 h of incubation at 30 °C and were used to inoculate 10 mL of CGIII medium. Cultivation of the isolated cells took place at 30 °C, 120 rpm in a non-baffled shake flask. After 20 h of cultivation, the culture broths were diluted to an OD of 0.6 using PBS with a final concentration of 500 µM DFHBI. After 10 min of incubation, the cell suspension was analyzed in an Arialll High-speed cell sorter using the settings listed in d). Of the 19 cultures cultivated, 4 showed an 1.5- to 2-fold increased fluorescence in comparison to the starting strain *C. glutamicum* ATCC 13032 *Δcg2273* pJC1-PF1-U1A-F30::broccoli/UUCG-TF1 .

### f) Extraction of the RNA of interest from the cells isolated in d)

Using the culture broths analyzed in e), 1x10⁹ cells from the four best performing strains were used for RNA extraction with the Monarch total RNA kit (New England Biolabs, Ipswich, MA, USA). The isolated RNA was analyzed using an Agilent Bioanalyzer 2100 (Agilent Technologies, Santa Clara, CA, USA). The increased fluorescence corresponded to an increased target RNA abundance per total RNA extracted.

**Table 1**

| Strain | Specific fluorescence relative to the control strain / target RNA titer relative to the control strain |
|---|---|
| ***C. glutamicum* ATCC 13032 Δcg2273 pJC1-PF1-U1 A-F30::broccoli/UUCG-TF1 (Control)** | 100%/100% |
| Mutant 1 | 227% / 220% |
| Mutant 2 | 181% / 185% |
| Mutant 3 | 171% / 174% |
| Mutant 4 | 152% / 149% |

### g) Using isolated strains to produce target RNA without tag

The plasmid pJC1-PF1-U1A-F30::broccoli/UUCG-TF1 was removed from the isolated strains using an adapted version of the transformation protocol of Tauch *et al.,* 2002 (Tauch, A., Kirchner, O., Löffler, B., Götker, S., Pühler, A., and Kalinowski, J. Efficient Electrotransformation of Corynebacterium diphtheriae with a Mini-Replicon Derived from the Corynebacterium glutamicum Plasmid pGA1. Curr. Microbiol. 2002;45, 362-367). Strains were made competent according to Tauch *et al.,* 2002 and electroporation was performed without addition of plasmid DNA. Following the regeneration according to the original protocol, the cells were diluted and spread on non-selective CGIII agar. Grown colonies were streaked on CGIII agar with 15 µg/mL kanamycin and non-selective CGIII agar. Successful removal of pJC1-PF1-U1A-F30::broccoli/UUCG-TF1 from kanamycin-sensitive cells was confirmed by colony PCR (no product with primer combination pJC1_check_f and pJC1_check_rev). The plasmid-free strains thus produced were transformed with pJC1-PF1-U1A-TF1 as described in b) to enable production of the target RNA without the tag consisting of the F30 scaffold and broccoli. Cultivation of the strains according to the description in e) and extraction and analysis of the produced RNA as described in f) confirmed increased target RNA production in comparison to the control strain *C. glutamicum* ATCC 13032 *Δcg2273* pJC1-PF1-U1A-TF1.

## Claims

1. A method for optimizing the production of a RNA sequence of interest in a cell, comprising the steps of:
a) introducing into a plurality of cells a vector capable of expressing a heterologous RNA of interest, wherein said RNA is tagged with a RNA tag comprising
- an aptamer capable of stabilizing a fluorophore and
- a scaffold capable of stabilizing the aptamer;
b) culturing the cells in a culture medium under conditions that allow expression of the heterologous RNA of interest;
c) adding said fluorophore to the culture medium;
d) identifying those cells that show the highest intensity of fluorescence and therefore a high expression of the RNA of interest.

2. A method for producing a RNA of interest, comprising the steps of
a) introducing into a plurality of cells a vector capable of expressing a heterologous RNA of interest, wherein said RNA is tagged with a RNA tag comprising an aptamer capable of stabilizing a fluorophore and a scaffold capable of stabilizing the aptamer;
b) culturing the cells in a culture medium under conditions that allow expression of the heterologous RNA of interest;
c) adding said fluorophore to the culture medium;
d) identifying and isolating those cells that show the highest intensity of fluorescence and therefore a high expression of the RNA of interest;
e) removing the first vector of step a) from the cells isolated in step d);
f) introducing a second vector capable of expressing the heterologous RNA of interest without the RNA tag into the cells obtained in step e);
g) producing the RNA of interest by culturing the cells obtained in step f).

3. The method according to claim 1 or 2, wherein the cell is a microbial cell, in particular a gram positive bacterial cell.

4. The method according to any of claims 1 to 3, wherein in step d) the cells showing the highest intensity of fluorescence are identified using flow cytometry.

5. The method according to any of claims 1 to 4, wherein the cells show different levels of expression of the heterologous RNA due to culture conditions, in particular temperature, pressure and/or culture medium, chromosomal genetic alterations or genetic alterations in the vector.

6. The method according to any of claims 1 to 5, wherein the aptamer comprises a sequence according to SEQ-ID No: 1 to 8, 13 to 16, 65 or 66.

7. The method according to any of claims 1 to 6, wherein the RNA scaffold capable of stabilizing the aptamer comprises the sequence according to SEQ-ID No: 9 to 12.

8. The method according to any of claims 1 to 7, wherein the RNA tag comprises a sequence according to SEQ-ID No: 17 to 64, 69, 77 or 78.

9. A cell harboring a vector capable of expressing a heterologous RNA of interest, wherein said RNA is tagged with a RNA tag comprising
- an aptamer capable of stabilizing a fluorophore and
- a RNA scaffold capable of stabilizing the aptamer.

10. The cell according to claim 9, wherein the cell is a microbial cell, in particular a gram positive bacterial cell.

11. The cell according to claim 10, wherein the gram positive bacterial cell is a cell from the genus *Corynebacterium,* in particular *Corynebacterium glutamicum.*

12. The cell according to any of claims 9 to 11, wherein the aptamer comprises a sequence according to SEQ-ID No: 1 to 8, 13 to 16, 65 or 66.

13. The cell according to any of claims 9 to 12, wherein the aptamer is capable of stabilizing any fluorophores selected from 2-HBI, DFHBI, DFHBI-1T, DFHBI-2T, DMABI or DMHBI, TO1, TO3 or Hoechst 1C.

14. The cell according to any of claims 9 to 13, wherein the RNA scaffold capable of stabilizing the aptamer comprises the sequence according to SEQ-ID No: 9 to 12.

15. The cell according to any of claims 9 to 14, wherein the RNA tag comprises a sequence according to SEQ-ID No: 17 to 64, 69, 77 or 78.
